Europäisches Patentamt

(19) European Patent Office

Office européen des brevets

(11) Publication number: **0 257 845 B1**

(12) # EUROPEAN PATENT SPECIFICATION

(45) Date of publication of patent specification:
16.10.91 Bulletin 91/42

(51) Int. Cl.⁵: **A01H 3/00, A01H 5/12**

(21) Application number: 87306856.3

(22) Date of filing: 03.08.87

(54) Method of producing variegated plants.

(30) Priority: 01.08.86 JP 180114/86
27.06.87 JP 158821/87

(43) Date of publication of application:
02.03.88 Bulletin 88/09

(45) Publication of the grant of the patent:
16.10.91 Bulletin 91/42

(84) Designated Contracting States:
CH DE FR GB IT LI NL SE

(56) References cited:
R. WEGLER: "CHEMIE DER PFLAN-
ZENSCHUTZ-UND
SCHÄDLINGSBEKÄMPFUNGSMITTEL", vol.
2, "Fungizide, Herbizide, Natürliche Pflanzen-
wuchsstoffe, Rückstandsprobleme", 1970,
pages 172-194, "Herbizide", Springer Verlag,
Berlin, DE;

(73) Proprietor: Taguchi, Masayasu
1321, Kishigawa-cho-kita
Naga-gun Wakayama-ken (JP)

(72) Inventor: Taguchi, Masayasu
1321, Kishigawa-cho-kita
Naga-gun Wakayama-ken (JP)

(74) Representative: Lamb, John Baxter et al
Reginald W. Barker & Co. 13, Charterhouse
Square
London EC1M 6BA (GB)

**Description**

BACKGROUND OF THE INVENTION

1. Field of the Invention

The present invention relates to a method for artificially producing variegation patterns in plants, in particular, in decorative plants, and more preferably, in foliage plants.

2. Description of the Prior Art

Among decorative plants, a large number of variegated plants have been known conventionally. These variegated plants are high in decorative value. Therefore, they are higher in demand for them and also higher in economical value than their green foliage type counterparts (plants without variegation). However, the conventional methods for producing such variegated foliage plantes are only those to obtain such plants through manifestation of genetic characters produced by natural mutation or artificial mutation, or those to obtain them through the contraction of disease by plants due to viruses.

Consequently, it has been difficult to industrially produce the variegated plants and mass produce them, and also, the plants obtained have generally been weaker in characteristics including the cold resistance, the light resistance and the resistance to blight and pest, in comparison with the green foliage types of the same species. Besides, they had the other problems in cultivation, such as the slow growing rate and the low breeding (reproduction) coefficient. Furthermore, in some cases due to the pathogenic germs of viruses, not only the malformation, such as the shrinkage of leaves,has been caused, but also the growing capability (viability) has been hampered, whereby degrading the commercial value of the plants.

SUMMARY OF THE INVENTION

The inventor of this invention found the following facts after conducting the strenuous studies for the purpose of solving the above mentioned problems in production of variegated plants.

More specifically, it was found that, through the use of a production method for obtaining various types of variegated plants (plants with tiger stripe color breaking, vertical color breaking, transverse color breaking, center portion color breaking, outer rim color breaking, speckles, vein speckles color breaking along vein, etc. as well as the whitening variegation, the yellowing variegation, etc.), which use specific chemicals (compounds) to be applied while utilizing the respective characteristics (veins, capability to absorb chemicals through leaf surface, etc.) of the respective plants during the growing process of the plants, the variegated plants with high ornamental value, which show the substantial difference in variegation pattern even among those belonging to the same family, genus, and species, can be obtained artificially.

As mentioned above, the mode (pattern) of color breaking may vary respectively in various types of plants, in many cases.

The following findings were also obtained. When some plants belonging to the same species are treated with various types of chrolophylls, respectively, not so remarkable difference distinguishing the modes of variegation among them is shown, but the details of the same mode of variegation may vary in aspects by showing the whitened color breaking, the yellowed color breaking, the color breaking with clear boundary between the variegated portion and the chrolophyll portion, the color breaking with mixed, splinkly boundary between the variegated portion and the chlorophyll portion, the light white and light yellow color breakings, the wide width color breaking, the narrow width color breaking, etc. Thus, it is possible to artificially produce variegated plants with different and highly ornamental value through the utilization of the respective properties of the various types of chlorophyll inhibitors (see the example of Rohdea described later as an embodiment).

In addition, it was confirmed that the variegated plants thus obtained are as strong and stout as the green leave plants in their cold resistance, light resistance, pest resistance, and other characteristics, as well as in their growth rate, breeding coefficient, etc. Also it easy to cultivate the thus obtained variegated plants. This invention was completed based upon the above studies.

Accordingly, the object of this invention is to provide a method for artificially producing variegated plants which have the characteristic aspects of color breakings, respectively, and which are the same as the conventional green leaf plants in their characteristics including the cold resistance, as well as in growth rate, etc., through treating various types of plants with various types of chlorophyll inhibitors by using various types of treating techniques.

2

## BRIEF DESCRIPTION OF THE DRAWINGS

Figures 1 through 6 illustrate the morphologic appearance (mode of the variegation) of the plants obtained by using the method provided by this invention, together with the morphology of the untreated plants shown for comparison. Figures 7 through 13 illustrate the morphology of the plants obtained by using the method according to this invention, wherein:

Figure 1(a) is a photograph showing variegated (tiger stripe color breaking) state of Rohdea (of Liliaceae) obtained by applying the method provided by this invention (Embodiment 1), and Figure 1(b) is a photograph showing the untreated state of the plant of the same species;

Figure 2(a) is a photograph showing the variegated (tiger stripe color breaking) state obtained by applying the method of this invention (Embodiment 2) to Yucca, yurehantipes (of Agavaceae), and Figure 2(b) is a photograph showing the untreated plant of the same species;

Figure 3(a) is a photograph showing the variegated (tiger stripe color breaking) state obtained by applying the method of this invention (Embodiment 3) to Dracaena fragrance (of Liliaceae), and Figure 3(b) is a photograph showing the untreated state thereof;

Figure 4(a) is a photograph showing the variegation (center portion color breaking) produced by applying the method of this invention (Embodiment 4) to Philoderdron oxycardium (of Araceae), and Figure 4(b) is a photograph showing the untreated state thereof;

Figure 5(a) is a photograph showing the variegated (transverse color breaking) state brought about by applying the method of this invention (Embodiment 27) to Dracaena fragrans (of Liliaceae), and Figure 5(b) is a photograph showing the untreated state thereof;

Figure 6(a) is a photograph showing the state of variegation (entire leaf whitening color breaking, center portion color breaking) obtained by applying the method of this invention (Embodiment 7) to Hong Kong Bombax, and Figure 6(b) is a photograph showing the untreated state thereof;

Figure 7 is a photograph showing the variegated (vertical color breaking) state obtained by applying the method of this invention (Embodiment 29) to Howela belmoreana (Coryphaceae);

Figure 8 is a photograph showing the variegated (outer rim portion color breaking) state obtained by applying the method of this invention (Embodiment 39) to Ebis Ardisia (Myrsinaceae);

Figure 9 is a photograph showing the variegated (center portion color breaking) state obtained by applying the method of this invention (Embodiment 53) to Peperomia (Piperaceae);

Figure 10 is a photograph showing the variegated (vein form color breaking) state obtained by applying the method of this invention (Embodiment 70) to Coffea (Rubiaceae);

Figure 11 is a photograph showing the variegated (vertical color breaking, tiger stripe color breaking) state obtained by applying the method of this invention (Embodiment 86) to bamboo (Bambusaceae);

Figure 12 is a photograph showing the variegated (dotted color breaking) state obtained by applying the method of this invention (Embodiment 87) to Equisetum (Equisetaceae);

Figure 13 shows the difference in manifestation of variegation amoung the embodiments on Rohdea (Liliaceae), in which:

Figure 13(a) is a photograph showing the medial state of variegation in which the boundary between the chlorophyll portion and the whitened portion is neither clear nor sprinkly, that is brought about by using 4-methoxy-3' .3-dimethylbenzophenone, by taking the same treating procedure as in Embodiment 1;

Figure 13(b) is a photograph showing the state of variegation in which the boundary between the chlorophyll portion and the yellowed portion is in sprinkly aspect, that is effected by using ammonium methanearsonate by the same method as in Embodiment 1; and

Figure 13(c) is a photograph showing the state in which the boundary between the chlorophyll portion and the yellowed portion is clearly observed, that results by applying 3.5. dichloro-2.6-difluoro-4-hydroxypyridine, by using the treating method same as that used in Embodiment 1.

## DETAILED DESCRIPTION OF THE INVENTION

Provided by the present invention is a method for producing variegated plants through destroying chlorophyll in the individual bodies of the target plants directly or indirectly, or through inhibiting the formation of chlorophyll by treating the foregoing plants with chlorophyll inhibitors, during the growing process of the plants.

The chlorophyll inhibitors in this invention are meant to be the substances which destroy the chlorophyll in individual target plants or which inhibit the formation of the chlorophyll in those individual plants during the growing process of the plants. Practical examples of such compounds are, for example, as follows.

4-methoxy-3.3'-dimethylbenzophenone

4-(2.4-dichlorobenzoyl) 1.3-dimethyl-1H-pyrazol-5-ylp-toluene sulfonate
3.4-dichlorobenzyl-N-methyl carbamate
Methyl N-(4-nitrophenylsulfonyl) carbamate
Methyl N-(4-aminophenylsulfonyl) carbamate
Isopropyl-N-(3-chlorophenyl) carbamate
2.3.5-trichloro-4-hydroxypyridine
3.5-dichloro-2.6-difluoro-4-hydroxypyridine
2-[4-(2,4-dichlorobenzoyl)-1.3-dimethylpyrazol-5-yloxy] acetophenone
4-chloro-5-methylamino-2-(3-trifluoromethylphenyl) pyridazin-3(2H)-one
4-chloro-5-dimethylamino-2-(3-trifluoromethylphenyl) pyridazin-3(2H)-one
3-amino-1H-1.2.4-triazole
1.2-dimethyl-3.5-diphenyl-1H-pyrazolium methyl sulfate
1-methyl-3-phenyl-5-(3-trifluoromethylphenyl)pyridin-4(1H)-one
2.4-dinitro-6-secondary butylphenyl dimethyl acrylate
Dinitromethylheptyl phenyl crotonate
1.1-bis(chlorophenyl)-2.2.2-trichloroethanol
Mixture of 0.0-disopropyl s-(2-phenylsulfonylaminoethyl) phosphorodithioate and 4-methoxy-3.3 dimethylbenzophenone
a,a,a trifluoro-2.6-dinitro-N,N-dipropyl-p-toluidine
0-methyl-0-(2-nitro-4-methylphenyl)-N-isopropyl-phosphoroamide thioate
4-(methylsulfonyl)-2.6-dinitro-N,N-dipropylaniline
Dichlorophenoxyacetic acid
Calcium trichloroacetate
2-methyl-4-chlorophenoxyacetic acid
2.4-dinitro-6-secondary butylphenol acetate
2.2-sodium dichloropropionate
a-(2-naphthoxy)propionanilide gibberellin
Ammonium methanearsonate
Sodium pyroborate
Sodium octaborate
Sodium metaborate
Sodium chlorate

The mechanisms of these compounds are to destroy the chlorophyll directly or indirectly or to inhibit the formation of the chlorophyll in the following ways. That is, for example, these compounds inhibit the biosynthesis of the chlorophyll itself; they inhibit the synthesis of chlorophyll through the excessive incorporation of boron; they obstruct the synthesis of chlorophyll through blocking the intake of magnesium that is the central metal of chlorophyll or through destroying proteins and amino acids; they prevent the synthesis of chlorophyll through the abnormal growth of cells, etc., by means of the hormonal activity; or they check the synthesis of clorophyll by inhibiting the formation of carotenoid that prevents the photolysis of the chlorophyll or by hampering the electronic transfer system.

More particularly, 4-(2.4-dichlorobenzoyl)-1.3-dimethyl-1H-pyrazol-5-yl p-toluene sulfonate; 3.4-dichlorobenzyl-N-methyl carbamate; 1.1-bis(chlorophenyl)-2.2.2-trichloro-ethanol; the mixture of 3% 0.0-disopropyl s-(2-phenylsulfonyl-aminoethyl) phosphorodithioate, 8% 4-methyoxy-3.3'-dimethylbenzophenone and 89% other mineral fines; calcium trichloroacetate, ammonium methanearsonate; sodium chlorate, etc. inhibits the biosynthesis of the chlorophyll itself.

Also, sodium tetraborate, sodium octaborate, sodium metaborate, etc. inhibits the biosynthesis of the chlorophyll through the excessive incorporation of boron, etc. into plant body, and 3-amino-1H-1.2.4-triazole obstructs the biosynthesis of the chlorophyll by inhibiting the intake of mangnesium, iron, manganese, etc.

Methyl N-(4-nitrophenylfulfonyl) carbamate, methyl N-(4-aminophenylsulfonyl) carbamate, 2.4-dinitro-6-secondary butyl-phenyldimethyl acrylate, dinitromethylheptylphenyl crotonate a,a,a-trifluoro-2.6-dinitro-N,N-dipropyl-p-toluidine, 4-(methylsulfonyl)-2.6-dinitro-N,N-dipropyl-aniline, 2.4-dinitro-6-secondary butylphenol acetate, etc. inhibits the biosynthesis of chlorophyll by destroying proteins and amino acids.

Furthermore, basic copper chloride prevents the formation of the chlorophyll by destroying proteins and amino acids by the copper ions.

3.5-dichloro-2.6-difluoro-4-hydroxypyridine, 0-methyl-0-(2-nitro-4-methylphenyl)-N-isopropylphosphoroa, 2-methyl-4-chlorophenoxyacetic acid, a-(2-naphtoxy) propionanilide, dichlorophenoxyacetic acid, gibberellin, etc. inhibit the biosynthesis of chlorophyll through the abnormal growth of cells, etc., by means of the hormonal activity.

Also, 2.2-sodium dichloropropionate and isopropyl-N-(3-chlorophenyl) carbamate respectively prevents the biosynthesis of chlorophyll by the destruction of proteins and amino acids as well as the abnormal growth of cells by means of the hormonal activity.

4-methoxy-3.3'-dimethylbenzophenone, 2-[4-(2.4-dichlorobenzoyl)-1.3-dimethylpyrazol-5-yloxyl] acetophenone, 4-chloro-5-methylamino-2-(3-trifluoromehtylphenyl) pyridazin-3(2H)-one, and 4-chloro-5-dimethylamino-2-(3-trifluoro-methylphenyll)pyridazin-3 (2H)-one, and 1-methyl-3-phenyl-5-(3-trifluoroethylphenyl) pyridin-4 (1H)-one respectively inhibit the formation of carotinoid that inhibits the photolysis of chlorophyll.

Furthermore, 2,3,5-trichloro-4-hydroxy-pyridine inhibits the synthesis of chlorophyll by blocking the biosynthesis of carotinoid and also by the abnormal growth of cells due to the hormonal activity.

In addition, 1.2-dimethyl-3.5-diphenyl-1H-pyrazolium-methyl sulfate inhibits the biosynthesis of chlorophyll by obstructing the electron transfer system.

The plants used in this invention are, for example, those belonging to Liliaceae,such as Rohdea, Dracaena, Asparagus, Sansevieria, and Cordyline; Agavaceae, such as Agave and Yucca; Araceae, such as Aglaonema, Philodendron, Anthurium, Spathiphyllum, Zantedeschia, Monstera, Alocasia, Caladium, Syngonium, Dieffenbachia, and Pothos; Myrsinaceae, such as Ardisia; Araliaceae, such as Ivy, Bombax, and Schefflera; Musaceae, such as Musa and Strelitzia; Bombacaceae, such as Pachira; Palmae (Coryphaceae), such as Rhapis excelsa, and Phoenix; Bromeliaceae, such as Ananas, Tillandsia, and Viresea carinata; Marantaceae, such as Calathea and Maranta; Moraceae, such as gum tree and Ficus; Euphorbiaceae, such as Codiaeum and Aleurites; Zingiberaceae, such as Hedychium and Alpinia; Orchidaceae, such as Orchid and Symbidium; Amaryllidaceae, such as Clivia miniata; Pandanaceae, such as Pandanus; Cyperaceae, such as Cyperus alternifolius; Polypodiaceae, such as Adina and Cacalia; Aspleniaceae, such as Mother Fern and Neottopteris; Marattiaceae, such as Angiopteris; Pteridaceae, such as Adiantum; Davalliaceae, such as Nephrolepis; Cyatheaceae, such as Cyathea mettenianaand Cyathea pudophylla; Dryopteraceae, such as Polystichopsis simplicior; Araucariaceae, such as Brazil pine; Cycadaceae, such as Cycas and Dioon; Cupressaceae, such as Gold Crest and Thuja occidentalis; Podocarpaceae, such as Podocarpus; Urticaceae, such as Pellionia deveauana; Nyctaginaceae, such as Bougainvillea; Sarcandraceae, such as Chloranthus and Sarcandra; Hypericaceae, such as Garcinia Subelliptica; Sarraceniaceae, such as Sarracenia alata and Sarracenia rubra; Begoniaceae, such as Begonia-oizumi; Asteraceae, such as Gynura and Senecio; Cactaceae, such as Epiphyllum pumilum; Schisandraceae, such as Kadsura; Lauraceae, such as Laurus; Piperaceae, such as Peperomia; Crassulaceae, such as Crassuli argentea; Saxifragaceae, such as Bauera rubioides and Saxifraga; Rutaceae, such as Melicopetriphylla; Aquifoliaceae, Such as English Holly; Vitaceae, such as parthenocissus tricuspidata; Sterculiaceae, such as Sterculia foetida; Passifloraceae, such as Passiflora edulis; Caricaceae, such as Carica and Carica pentagona; Myrtaceae, such as Feijoa; Melastomaceae,such as Miconia; Rhizophoraceae, such as Bruguiera Gymnorrhiza and Kandellia; Apocynaceae, such as Pachypodium lamerei; Asclepiadaceae, such as Hoya; Rubiaceae, such as Psychotria serpens; Verbenaceae, such as Clerodendron; Lamiaceae, such as Orchosiphon aristalos; Bignoniaceae, such as Stereospermum; Acanthaceae, such as Repanda; Gesneriaceae, such as Obconicus; Caprifoliaceae, such as gomoju; Campanulaceae, such as Lobelia boninensis; Goodeniaccac, such as Scaevola; Iridaceae, such as American Iris; Commelinaceae, such as Callisia repens; Gramineae (Poaceae), such as Phragmites communis, Oplisonenus hiytellus and barley, wheat. etc.; Bambusaceae, such as bamboo; Equisetaceae, such as Equisetum; Primulaceae, such as Cyclamen; Pittosporaceae, such as Sharinbai; Oak in Fagaceae, such as Babe (Japanese); Gentianaceae such as Gentiana (Japanese); Pinaceae, such as Pinus; Buxaceae, such as Buxus; and Myricaceae, such as Myrica.

When the present invention is applied to the above-identified plants, particularly satisfactory results of variegation can be obtained, that is, the whitening or yellowing in color breaking is shown clearly. Besides, the state of color breaking resulted is stable. Thus, the variegated plants with high decorative value can be obtained from the foregoing plants.

In this invention, the above mentioned compounds may be used as a single type variegated plant producing agent for plants, respectively, or they may be used together with the other agents or materials, such as surface active agent, dissolution accelerating agent, extending agent, spreading agent, or solidification agent, which may be added within the range not to substantially obstruct the previously mentioned actions of the compounds. They may be used in a form of liquid agent (aqueous solution, solution with agents dispersed in water, etc.), powder agent (clay, China clay, Kaolin, diatomaceous earth, etc. are used), granular agent (particles, etc.), etc. The content (the amount of the effective components in the agent) of the foregoing compounds (effective components) in the variegated plant producing agent (that is, chlorophyll inhibitor) is about 10-100%-, and these compounds are used as they are or by after being diluted, depending on the form of agent. The amount of the effective components used for the foliage plant is, preferably, 0.0001 to 100,000 mg per kg of the weight of the plant, and under such condition, the particularly satisfactory variegation can be resulted.

The variegated plant producing agent may be used at any time as long as it is in the growing process of the target plant. However, the germinal stage or the leaf growing stage are preferable, and particularly the leaf growing stage is preferable. Any portion of the target plant can be treated with the variegated plant producing agent. For example, the seed, bud, leaf, stem, root, fruit, tuber, or rhizome, hut the bud, leaf, stem or root is specifically preferable in view of the absorption of the effective components.

As to the method for treating the plant with the variegated plant producing agent, the agent may be sprayed onto the leaf surface in a form of liquid or powder. It may be poured (sprinkled) onto the root and the surface of soil so that the agent permeates into the soil, that is, it may be given by the irrigation of soil. The agent may be injected, by using a syringe, into the plant body, such as the bud region, or into the soil around the area having the largest number of growing-out roots.

Furthermore, the foregoing agent may be buried, in a form of particle or powder, into the soil or the medium instead of soil, at the root of the plant.

The relationship between the respective methods for treating potted plants and outdoor plants with the compounds as the modes of variegation is as follows. With respect to the potted plants, when they are treated by dispersion (spraying), the boundary line between the chlorophyll portion and the variagated portion becomes zigzagged with light color breaking shown as a whole. In this case, it frequently happens that chlorophyll in cloud-shape, dotted-form, vertical form, etc. remains in the variegated portion, thus showing the interesting manifestation of color breaking. On the other hand, when the treatment is performed by burying, irrigation or injection, the boundary line between the clorophyll portion and the variegated portion appears as a single straight line, and the admirably variegated plants with wide variegated portion and with almost no chlorophyll remaining in the variegated portion can be obtained.

With regard to the outdoor plants, when they are treated by dispersion (spraying), the boundary line between the chlorophyll portion and the variegated portion become zigzagged, with light colored variegation resulting as a whole, and the chlorophyll in cloud-shape, dotted form, vertical shape, etc. remains in the variegated portion, thus showing the interesting manifestation of color breaking. When the plants are treated with irrigation or burial of agents, because of the large amount of the soil, only a small dosage of the agent is absorbed into the plant body, and the variegation of the chic and stable apearance is resulted. When the treatment is given by injection, since the large dosage can be taken into the plant body, the boundary line between the chlorophyll portion and the variegated portion appears as a single straight line, and an excellent variegation with a wide color breading portion and with almost no chlorophyll remaining in the variegated portion can be achieved.

As mentioned above, depending upon the difference between the potted plants and the outdoor plants, and also by the difference among the treating methods, such as spraying, injection, irrigation and burying, the modes of variegation having the substantial differences respectively can be created artificially.

The time until the variegation of the plants treated as mentioned above becomes manisfested and stabilized, as well as the mode of the variegation, differs depending on the family, genus, species, variety of the plants, treating method, and condition for cultivation, when the cultivation is continued by using the ordinary cultivation method after the treatment. However, the time taken until the variegation is stabilized is about 10 to 50 days, and usually, approximately on the 10th to 50th day after the treatment, the stable and satisfactory variegation is effected.

As to the mode of the variegation, through the use of the producing methods for various types of variegations, which utilize the characteristics of the respective types of plants, such as the veins and the capability of absorbing the chemicals from leaf surface, the variegation modes which are substantially different even among the fellow families, genera and species, and which are high in ornamental value, respectively, can be obtained artificially. The foregoing modes of variegation include the tiger stripe color breaking, vertical color breaking, transverse color breaking, center (middle) portion color breaking, outer rim portion color breaking, dotted color breaking, vein-form color breaking, whitening variegation, and yellowing variegation.

The effective components used in this invention, for example, 4-methoxy-3.3'-dixethylhenzophenone and 4-(2.4-dichlorobenzoyl)-1.3 dimethyl-1H-pyrazol-5-yl p-toluene sulfonate, are the compounds which usually wither the plants. The purpose of the use of these chemicals is to kill the plants by withering, and the technique of using such chemicals for the other purpose, particularly, as in the present invention, for the purpose of producing the variegated plants with high ornamental value, without withering the plants or without stunting their growth, has not been known at all.

Also, the most of the effective components used in this invention are considered to bring the plants to wither to death when they are used by the methods which are usually employed by the people doing business in this industrial field. Besides, the effects of those effective components are selectively shown depending on the types of plants.

Furthermore, in the present invention, the purpose of the application of such effective components is not to eradicate the weeds intended to wither and eliminate, or to extirpate insects and pathogenic bacteria, but to produce the variegated plants and to grow them. Therefore, the scope of application of this invention is clearly different from those conventional ones. In addition, the application of the chemicals used in this invention to the purpose other than the purposes of whithering weeds, killing insects and germs, and controlling the growth of plants, is beyond the possibility for the people doing business in this industrial field to conceive it.

According to the methods provided by this invention, the plants variegated into having the same form of color breaking that is characteritic of each species can be obtained securely with high reproducibility by mass production. Besides, the viriegated plants obtained show absolutely no difference from those before the treatment or the green leaf types of the same species in their characteristics including the environment resistance, such as the cold resistance and light resistance, as well as the blight and pest resistance, the growth rate, and the breeding (reproduction) rate, and they can be cultivated with no difficulty. Furthermore, the variegated plants thus produced are superior to the conventionally known variegated plants which are obtained by the genetic reason or due to the action of viruses, in terms of the above mentioned characteristics. Therefore, they are extremely useful in industrial application.

In other words, generally, the genetically produced variegated plants are, as mentioned previously, inferior to the green leaf types in the resistance to environment, such as the disease resistance and light resistance. Besides, they are low in growth rate, and it is difficult to control their cultivation as well as to breed them. As a result, various types of difficulties are involved in their mass cultivation.

Due to virues, the variegated plants naturally suffer malformation, such as the shrinkage of leaves, due to the pathogenic toxicants carried by viruses. Thus, they are not high also in the commercial value.

## EMBODIMENTS

Hereunder, the embodiments will be shown. In these embodiments, the growth rate is indicated by the valve obtained by measuring, with the eye, the degree of growth in the case where the plant is cultivated under the conditions same as those for the untreated plant, by setting that the degree of growth of the untreated plant is 100.

### Embodiment 1

The variegated foliage plant producing agent was prepared by suspending, by dispersion, 100g of powder-form agent composed of 50% in 4-methoxy-3.3'-dimethylbenzophenone and 50% in mineral fines, and 10 ml of polyoxyethylene alkyl ether [surface active agent from Takeda Chemical Industries, Ltd., the trade name is "Alsope 30'"), in 10 l of water.

The agent thus prepared was sprayed (sprinkled), in amount of 10 ml (the amount of the effective components sprinkled per kg of body weight of plant was 125 mg), to Rohdea (plant of Liliaceae) of 0.4 kg in body weight and in the third year after the germination.

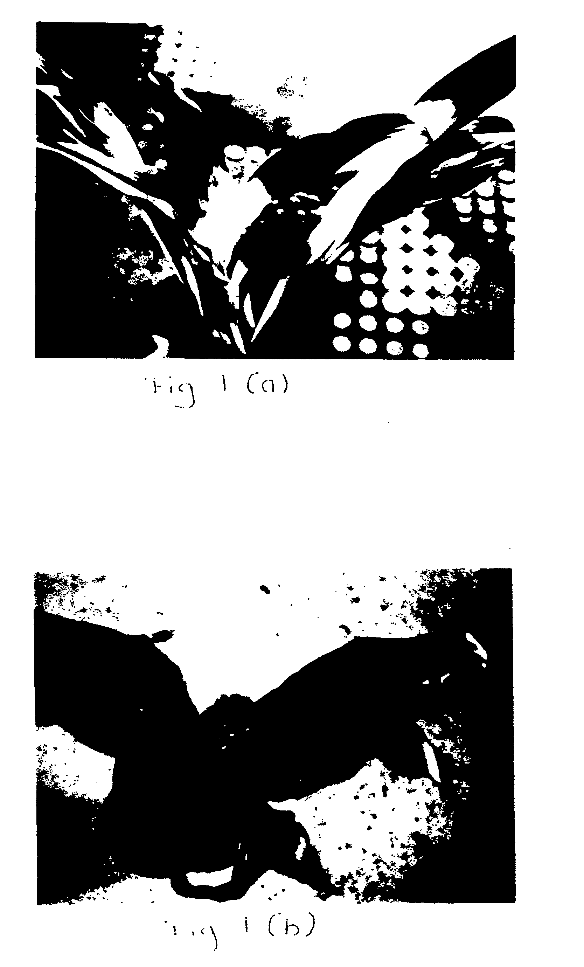

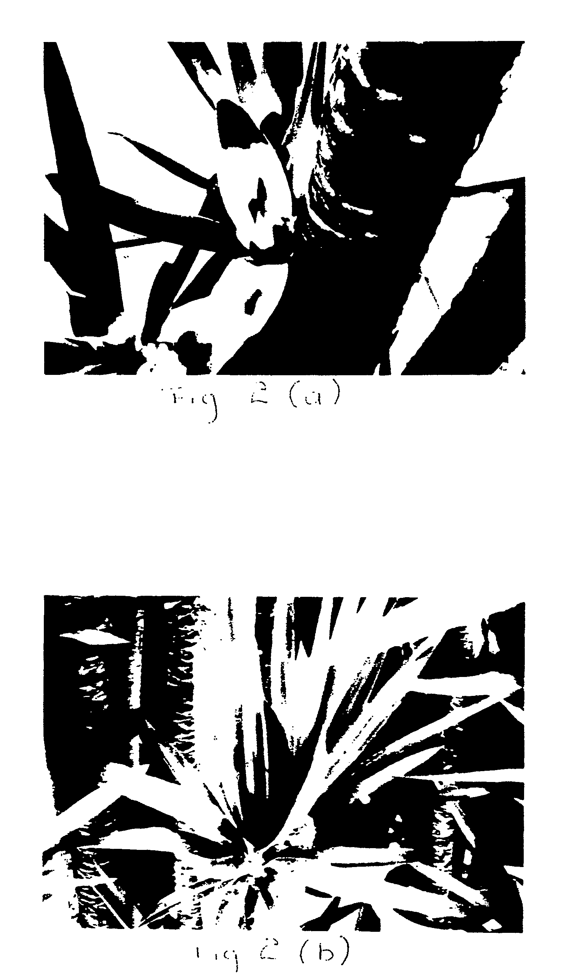

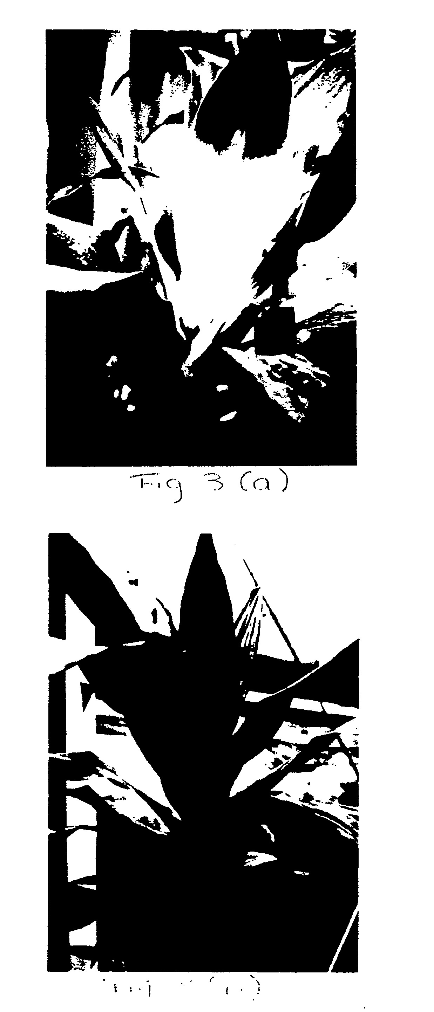

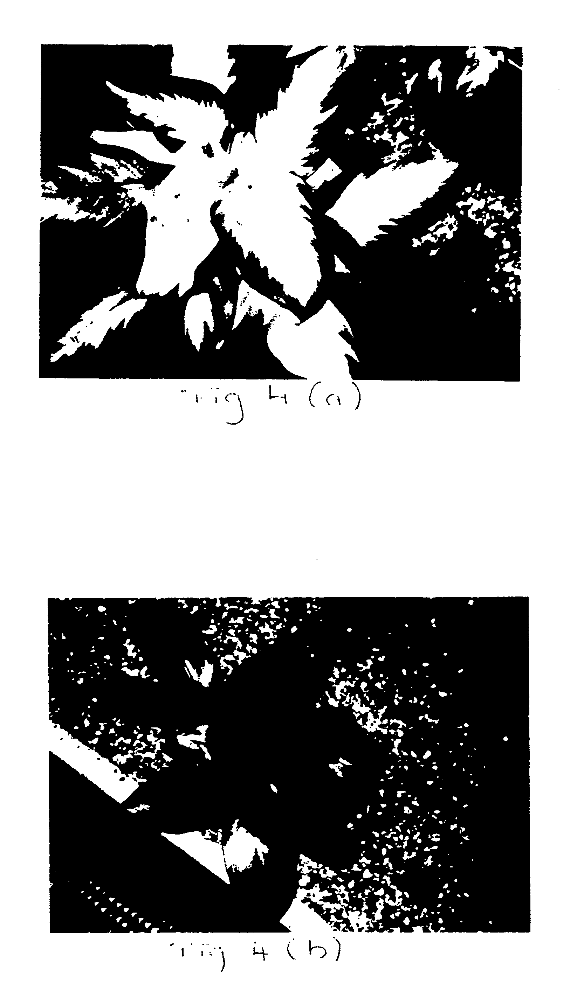

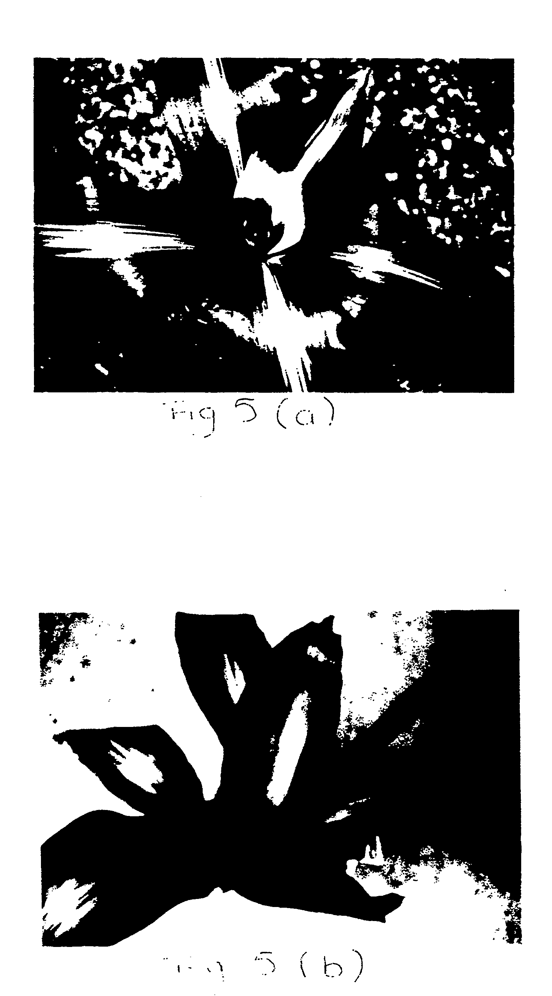

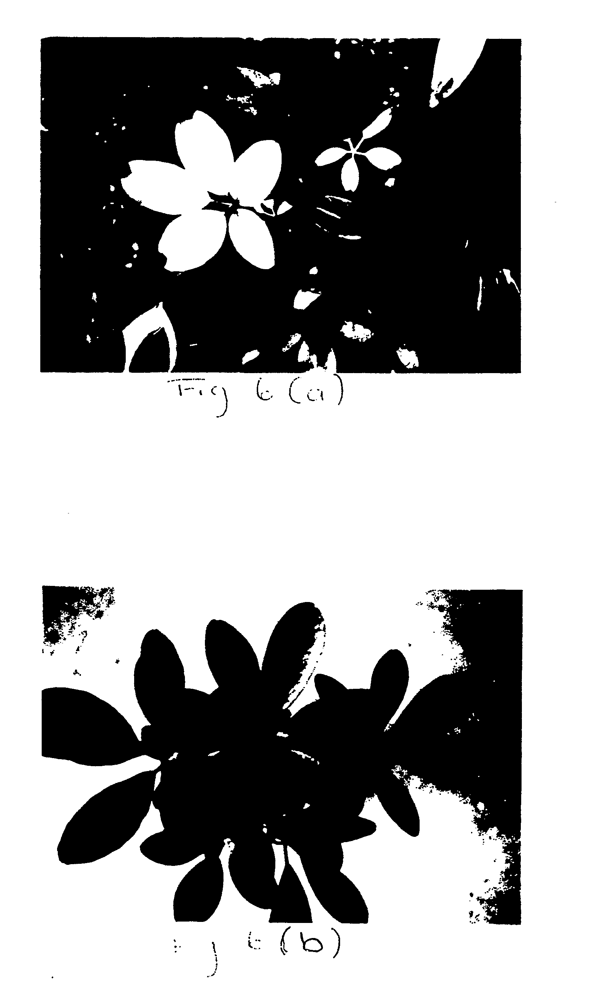

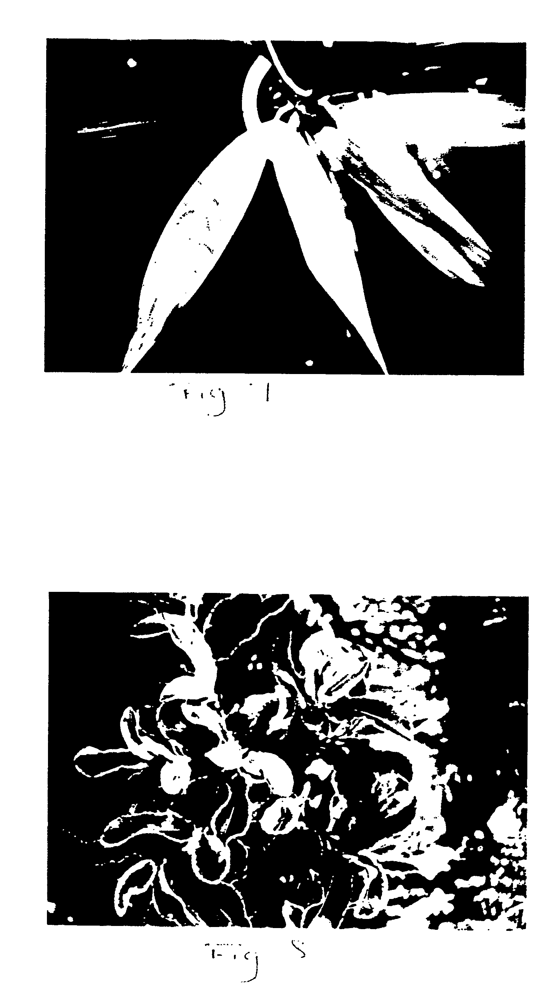

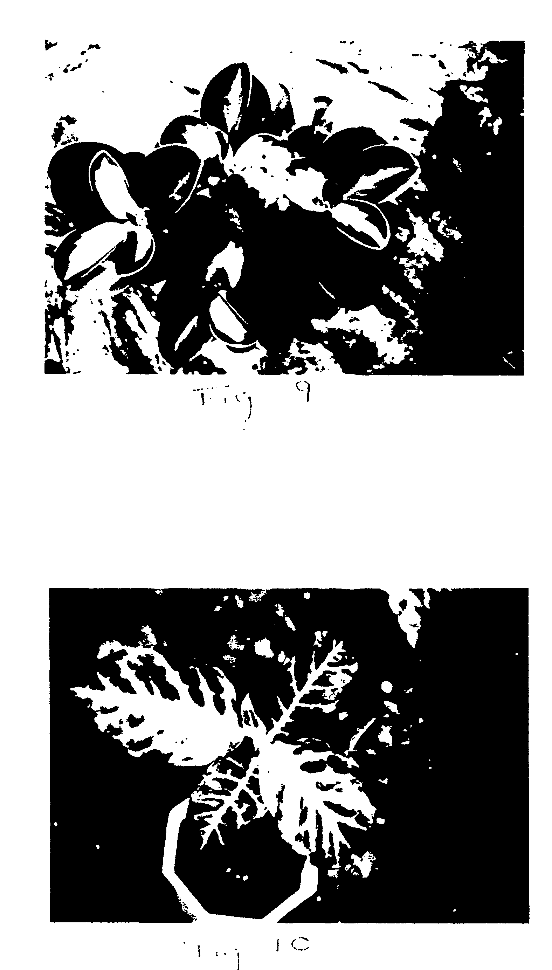

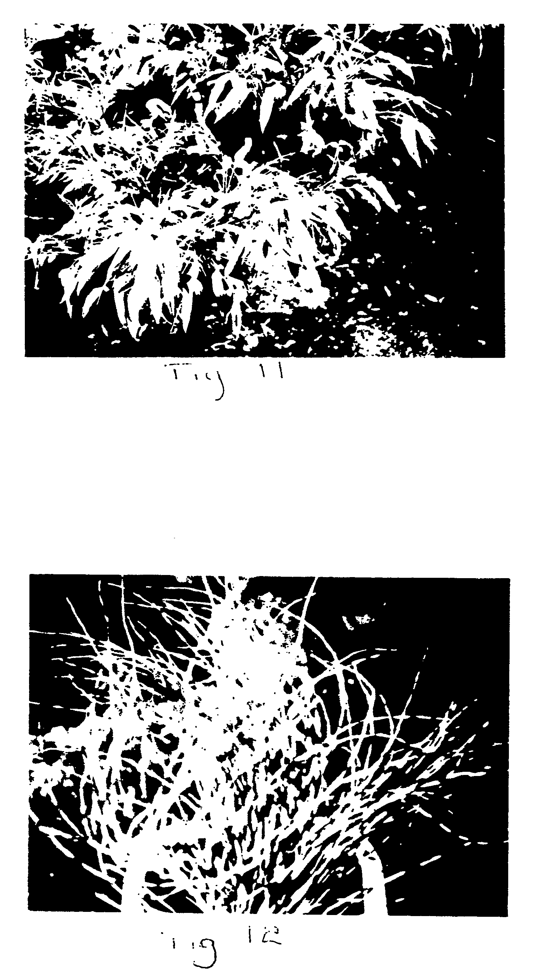

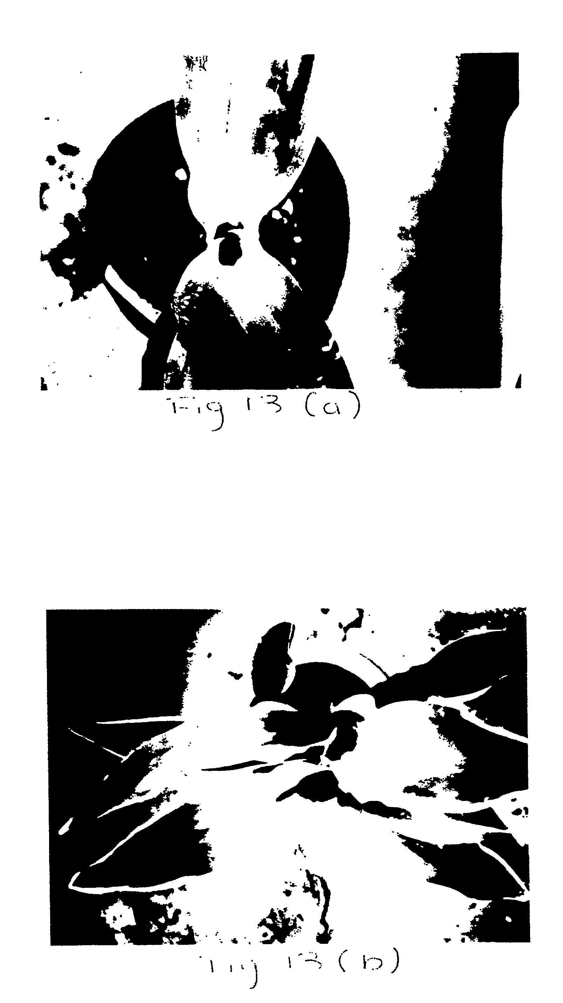

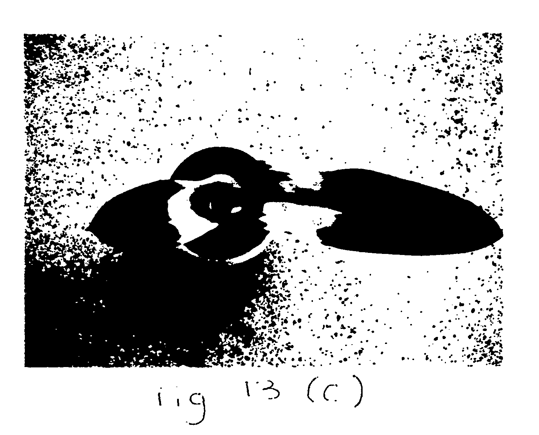

After the sprinkling, the color breaking appeared on the 14th day. The variegated state on the 50th day after the sprinkling is shown in Figure 1(a) (photograph). Also, the state of the control sample plant without treated by the sprinkling of the executive components is shown in Figure 1(b), for comparison.

As should be apparent from the Figure, by the application of this invention, the color breaking with good appearance was effected, and the variegation thus manifested was stable.

Furthermore, the variegated Rohdea thus obtained was same as the green leaf type (control group), in its characteristics including the environment resistance, blight and pest resistance, and breeding rate.

### Embodiment 2

The variegated foliage plant producing agent was prepared by mixing 10% in 4-methoxy-3.3'-dimethylbenzophenone with 90% in mineral fines, then by forming the mixture into grains.

The agent thus prepared was buried into the soil at the root of Yucca yurehantipes (plant of Agavaceae) of 0.5 kg in body weight in the leaf growing stage in the third month after planting the cutting. The amount of the agent buried in the soil was 20 mg/cm$^2$ [2000 mg in effective components per Kg of the body weight of the plant].

The color breaking was started on the 30th day after the burying was done, and the stable and satisfactory variegation was obtained after 50 days.

The variegated plants thus obtained were same as the green leaf type plants (control group) in their characteristics.

### Embodiment 3

0.5 g of 4-methoxy-3.3′-dimethylbenzophenone and 10 ml of surface active agent ("Alsope 30′" ® from Takeda Chemical Industries, Ltd.) were dispersed and suspended in 10 l of water. In this manner, the variegated foliage plant producing agent was prepared.

0.4 ml of the agent thus prepared was sprinkled (the amount of the effective components sprinkled per kg of the body weight of the plant was 0.1 mg) to Dracaena fragrance (plant of Liliaceae) of 0.2 kg in body weight in the third year of planting the cutting.

After the sprinkling, the color breaking was manifested on the 10th day. This color breaking was stable even after this plant grew and became satisfactory in appearance as variegation. Also, the variegated plants thus obtained were equivalent to the green leaf type counterparts in control group in their characteristics.

### Embodiments 4 through 95

The variegated plant producing agents were prepared by using the chemicals having the effective components shown in the applicable columns of Table 1. Then, the agents thus prepared were applied to the respective foliage plants shown in the same Table by using the treatment methods and dosages for treatment listed in the Table.

As a result, after several tens days, the stable variegation with good appearance were effected. Furthermore, these variegated plants were similar to their green leaf type counterparts (untreated control group plants) in their characteristics.

| Embodiment | Name of Plant (Family Name) | Name of Effective Component |
|---|---|---|
| 4 | Pxilodendron Oxycardium (Araceae) | 4-methoxy-3.3'-dimethylbenzophenone |
| 5 | Ardisia (Myrsinaceae) | Same as ahove |
| 6 | Spathiphyllum Patinie (Araceae) | Same as above |
| 7 | Schefflera (Hong Kong Bombax) (Araliaceae) | Same as above |
| 8 | Musa (Musaceae) | Same as above |
| 9 | Benjamin Gum tree (Moraceae) | Same as ahove |
| 10 | Pachira (Bombacaceae) | Same as above |
| 11 | Anthurium (Araceae) | Same as above |
| 12 | Rhpis excelsa (Coryphaceae) | 4-methoxy-3,3'-dimethylbenzophenone |
| 13 | Codiaeum (Euphorbiaceae) | 3.4-dichlorobenzyl-N-methylcarbamate |
| 14 | Same as above | Methyl N-(4-aminophenylsulfonyl) carbamate |
| 15 | Hedychium (Zingiberaceae) | 2.3.5-trichloro-4-hydroxypyridine |
| 16 | Same as above | 3.5-dichloro-2.6-difluoro-4-hydroxypyridine |
| 17 | Cymbidium (Orchidaceae) | 4-methoxy-3.3'-dimethylbenzo-phenone |
| 18 | Same as above | Same as above |
| 19 | Clivia miniata (Amaryllidaceae) | Ammonium methaneersonate |

| Embodiment | Name of Plant (Family Name) | Name of Effective Component |
|---|---|---|
| 20 | Clivia miniata (Amaryllidaceae) | Sodium tetraborate |
| 21 | Pandanus (Pandanaceae) | Sodium octaborate |
| 22 | Same as above | Sodium metaborate |
| 23 | Cyperus alternifolio (Cyperaceae) | 3-amino-1H-1.2.4-triazole |
| 24 | Adiantum (Pteridaceae) | 1.2-dimethyl-3.5-diphenyl-1H-Pyrazoliummethyl sulfate |
| 25 | Tillandsia (Bromeliaceae) | 2-[4-2.4-dichlorobenzoyl) 1.3-dimethylpyrazol-5-yloxy] acetophenone |
| 26 | Calathea lancifolia (Marantaceae) | 1-methyl-3-phenyl-5-(3-trifluoromethylphenyl) pyridin-4-(1H)-one |
| 27 | Dracaena fragrans (Liliaceae) | 4-methoxy-3.3'-dimethylbenzophenone |
| 28 | Dracaena concinna (Liliaceae) | 4-methoxy-3.3'-dimethyibenzophenone |
| 29 | Howela belmoreana (Coryphaceae) | Same as ahove |
| 30 | Bubaldia (Rubiaceae) | 1.1-bis (chlorophenyl)-2.2.2-trichloroethanol |
| 31 | Neottopteris nidus (Aspleniaceae) | Sodium chlorate |
| 32 | Angiopteris (Marattiaceae) | Basic copper chloride |
| 33 | Adiantum (Pteridaceae) | Mixture of 3% 0.0-disopropyl S-(2-phenylsulfonylaminoethyl) Phosphorodithioate, 8% 4-methoxy-3.3'-dimethylbenzophenone, and 89% mineral fines |
| 34 | Nephrolepis (Davalliaceae) | a,a,a-trifluoro-2.6-dinitro-N,N-dipropyl-p-toluidine |

| Embodiment | Name of Plant (Family Name) | Name of Effective Component |
|---|---|---|
| 35 | Cyathea metteniana (Cyatheaceae) | O-methyl-O-(2-nitro-4-xethyl-phenyl)-N-isopropylphosphoro-amidothioate |
| 36 | Polystichopsis simplicior (Dryopteraceae) | 4-(methylsulfonyl)-2.6-dinitro-N,N-dipropylaniline |
| 37 | Brazil pine (Araucariaceae) | Calcium trichloroacetate |
| 38 | Cycas (Cycadaceae) | 3.5-dichloro-2.6-difluoro-4-hydroxypyridine |
| 39 | Ebisu ardisia (Myrsinaceae) | Gibberellin |
| 40 | Podocarpus (Podocarpaceae) | 2-methyl-4-chlorophenoxyacetic acid |
| 41 | Pellinoia daveanana (Urticaceae) | 2.2-sodium dichloropropionate |
| 42 | Bougainvillea (Nyctaginaceae) | a-(2-naphthoxy)propionanilide |
| 43 | Chloranthus spicatus (Sarcandraceae) | Dichlorophenoxyacetic acid |
| 44 | Garcinia subelliptica (Hypericaceae) | Isopropyl-N-(3-chlorophenyl)carbamate |
| 45 | Dracaena surculosa (Liliaceae) | 4-methoxy-3.3'-dimethyl-benzophenone |
| 46 | Sarracenia minor (Sarraceniaceae) | 2.4-dinitro-6-secondary butylphenolacetate |
| 47 | Begonia oizumi (Begoniaceae) | 2-methyl-4-chlorophenoxy-acetic acid |
| 48 | Dracaena fragrans (Liliaceae) | 2.3.5-trichlcro-4-hydroxy-pyridine |
| 49 | Gynura (Asteraceae) | 2.2-sodium dichloropropionate |

11

| Embodiment | Name of Plant (Family Name) | Name of Effective Component |
|---|---|---|
| 50 | Epiphyllum pumilum (Cactaceae) | a-(2-naphthoxy) propionanilide |
| 51 | Kadsura (Schisandraceae) | Dichlorophenoxyacetic acid |
| 52 | Laurus (Lauraceae) | Isopropyl-N-(3-chlorophenyl) carbamate |
| 53 | Peperomia (Piperaceae) | 3.5-dichloro-2-6-difluoro-4-hydroxypyridine |
| 54 | Crassula argentea (Crassulaceae) | Dinitromethylheptyiphenyl crotonate |
| 55 | Saxifraga (Saxifragaceae) | 2.2-sodium dichloropropionate |
| 56 | Melicope triphylla (Rutaceae) | a-(2-naphthoxy) propionanilide |
| 57 | Dracaena concinna (Liliaceae) | Ammonium methanearsonate |
| 58 | English Holly (Aquifoliaceae) | Dichlorophenoxyacetic acid |
| 59 | Parthenocissus tricuspidata (Vitaceae) | Isopropyl-N-(3-chlorophenyl) carbamate |
| 60 | Sterculia foetida (Sterculiaceae) | 2.4-dinitro-6-secondary butyl-phenyldimethyl acrylate |
| 61 | Dracaena surculosa (Liliaceae) | Ammonium methanearsonate |
| 62 | Passiflora edulis (Passifloraceae) | 2-methyl-4-chlorophenoxy-acetic acid |
| 63 | Carica pentagona (Caricaceae) | 2.2-sodium dichloropropionate |
| 64 | Feijoa (Myrtaceae) | a-(2-naphthoxy)propionanilide |

| Embodiment | Name of Plant (Family Name) | Name of Effective Component |
|---|---|---|
| 65 | Miconia (Melastomaceae) | Dichlorophenoxyacetic acid |
| 66 | Bruguiera gymnorrhiza (Rhizophoraceae) | Isopropyl-N-(3-chlorophenyl) carbamate |
| 67 | Adenium obesum (Apocynaceae) | 2.2-sodium dichloropropionate |
| 68 | Asparagoides (Liliaceae) | 4-chloro-5 methylamino-2-(3-trifluoromethylphenyl) pyridazin-3(2H)-one |
| 69 | Asclepias fruticosa (Asclepiadaceae) | 2-[4-(2.4-dichlorobenzoyl)-1.3-dimethylpyrazol-5 yloxy] aceto-phenone |
| 70 | Coffea (Rubiaceae) | 4-methoxy-3.3'-dimethylbenzo-phenone |
| 71 | Kalanchoe tubiflora (Crassulaceae) | 2-methyl-4-chlorophenoxyacetic acid |
| 72 | Rhaps exelsa (Coryphaceae) | 4-methoxy-3.3'-dimethylbenzo-phenone |
| 73 | Clerodendrum philippinnon (Verbenaceae) | 2.2-sodium dichloropropionate |
| 74 | Orthosiphon aristalos (Lamiaceae) | a-(2-naphthoxy) propionanilide |
| 75 | Stereospermum (Bignoniaceae) | Dichlorophenoxyacetic acid |
| 76 | Hemigraphis (Acanthaceae) | 4-methoxy-3.3'-dimethyl-benzophenone |
| 77 | Aeschynanthus obconicus (Gesneriaceae) | Isopropyl-N-(3-chlorophenyl) carbamate |
| 78 | Viburnnm suspension | 2-[4-(2.4-dichlorobenzoyl)-1.3-dimethylpyrazol-5 yloxyl acetophenone |

13

## EP 0 257 845 B1

| Embodiment | Name of Plant (Family Name) | Name of Effective Component |
| --- | --- | --- |
| 79 | Lobelia boninensis (Campanulaceae) | 2-methyl-4-chlorophenolyacetic acid |
| 80 | Scaevola (Goodeniaceae) | 2-[4-(2.4-dichlorobenzoyl)-1.3-dimethylpyrazol-5 yloxy] acetophenone |
| 81 | American Iris (Iridaceae) | 2-methyl-4-chlorophenoxyacetic acid |
| 82 | Callisia repens (Commelinaceae) | 4-(2.4-dichlorobenzoyl)-1.3-dimethyl-1H-pyrazol-5 yl p-toluene sulfonate |
| 83 | Gold Crest (Cupressaceae) | 4-methoxy-3.3'-dimethyl-benzophenone |
| 84 | Barley, wheat, etc. (Gramineae) | 4-(2.4-dichlorobenzoyl)-1.3-dimethyl-1H-pyrazol-5 yl p-toluene sulfonate |
| 85 | Musa (Musaceae) | 4-methoxy-3.3'-dimethylbenzo-phenne |
| 86 | Bamboo (Bambusaceae) | Same as above |
| 87 | Equisetum (Equisetaceae) | Same as above |
| 88 | Gardenia (Rubiaceae) | 4-(2.4-dichlorobenzoyl)-1.3-dimethyl-1H-pyrazol-5 yl p-toluene sulfonate |
| 89 | Cyclamen (Primulaceae) | Same as above |
| 90 | Sharinbai (Japanese) (Pittosporaceae) | 4-methoxy-3.3'-dimethyl-benzophenone |
| 91 | Babe (Japanese) (Oak) | Same as above |
| 92 | Gentiana (Gentianaceae) | Same as above |
| 93 | Pinus (Pinaceae) | Same as above |

| Embodiment | Name of Plant (Family Name) | Name of Effective Component |
|---|---|---|
| 94 | Buxus (Buxaceae) | 2-(4-(2.4-dichlorobenzoyl)-1.3-dimethylpyrazol-5 yloxyl aceto-phenone |
| 95 | Myrica (Myricaceae) | 4-methoxy-3.3'-dimethyl-benzonhenone |

| Embodiment | Treating Period | Treated Region | Treating Method |
|---|---|---|---|
| 4 | Leaf growing stage | Root | Irrigation |
| 5 | " | Leaf | Spray |
| 6 | Germination stage | Germ | " |
| 7 | Leaf growing stage | Germ, Stem | Stem Injection |
| 8 | " | Leaf, Stem | Spray |
| 9 | " | " | " |
| 10 | " | Leaf | " |
| 11 | " | " | " |
| 12 | " | Leaf, Stem | " |
| 13 | " | " | Powder Spray |
| 14 | " | " | Spray |
| 15 | " | Leaf | " |
| 16 | " | " | " |
| 17 | Germination stage | " | " |
| 18 | " | " | " |
| 19 | " | " | " |
| 20 | " | Root | Burying |
| 21 | Leaf growing stage | " | " |
| 22 | " | " | " |
| 23 | " | Leaf | Spray |
| 24 | " | Stem, Leaf | " |
| 25 | Germination stage | Germ | Injection |
| 26 | Leaf growing stage | Leaf | Spray |
| 27 | " | " | " |
| 28 | Germination stage | " | " |
| 29 | " | " | " |

| Embodiment | Treating Period | Treated Region | Treating Method |
|---|---|---|---|
| 30 | Leaf growing stage | " | " |
| 31 | Germination stage | Germ | " |
| 32 | Leaf growing stage | Leaf | " |
| 33 | " | " | " |
| 34 | " | " | " |
| 35 | Germination stage | Root | Irrigation |
| 36 | Leaf growing stage | Leaf | Spray |
| 37 | Germination stage | Root | Irrigation |
| 38 | " | Germ | Spray |
| 39 | Leaf growing stage | Leaf | " |
| 40 | Germination stage | Germ | " |
| 41 | Leaf growing stage | Leaf | " |
| 42 | " | " | " |
| 43 | " | " | " |
| 44 | " | Root | Irrigation |
| 45 | " | Leaf | Spray |
| 46 | " | " | " |
| 47 | " | " | " |
| 48 | " | " | " |
| 49 | " | " | " |
| 50 | " | Root | Irrigation |
| 51 | " | " | " |
| 52 | " | Leaf | Spray |
| 53 | " | " | " |
| 54 | " | " | " |
| 55 | " | " | " |

| Embodiment | Treating Period | Treated Region | Treating Method |
|---|---|---|---|
| 56 | " | " | " |
| 57 | " | " | " |
| 58 | " | Root | Injection |
| 59 | Germination stage | " | Irrigation |
| 60 | " | Germ | Spray |
| 61 | Leaf growing stage | Leaf | " |
| 62 | " | " | " |
| 63 | " | " | " |
| 64 | " | " | " |
| 65 | " | " | " |
| 66 | " | " | " |
| 67 | " | " | " |
| 68 | Germination stage | Root | Irrigation |
| 69 | " | Germ | Spray |
| 70 | " | Leaf, Root | " |
| 71 | Leaf growing stage | Leaf | " |
| 72 | " | Root | Irrigation |
| 73 | " | Leaf | Spray |
| 74 | " | " | " |
| 75 | " | " | " |
| 76 | " | " | " |
| 77 | " | " | " |
| 78 | Germination stage | Root | Irrigation |
| 79 | " | Germ | Spray |
| 80 | Leaf growing stage | Leaf | " |
| 81 | " | " | " |

| Embodiment | Treating Period | Treated Region | Treating Method |
|---|---|---|---|
| 82 | " | " | " |
| 83 | " | " | " |
| 84 | Germination stage | Root | Irrigation |
| 85 | " | Germ | Spray |
| 86 | " | Leaf | " |
| 87 | " | " | " |
| 88 | Leaf growing stage | " | " |
| 89 | " | " | " |
| 90 | " | Root | Irrigation |
| 91 | " | Leaf | Spray |
| 92 | Germination stage | " | " |
| 93 | " | " | " |
| 94 | . " | " | " |
| 95 | Leaf growing stage | " | " |

| Embodiment | Dilution Ratio | Dosage/Kg of Body Weight of Plant (mg) | Mode of Variegation | Growth Rate |
|---|---|---|---|---|
| 4 | 100,000 | 0.1 | Center part color breaking | 98 |
| 5 | 100 | 100 | Outer rim variegation Center part variegation Tiger stripe variegation Entire leaf whitening | 98 |
| 6 | 3,000 | 0.3 | Tiger stripe variegation Vertical color breaking | 91 |
| 7 | 30 | 20 | Entire leaf whitening Center part variegation | 98 |
| 8 | 100 | 6 | Tiger stripe pattern | 97 |
| 9 | 20 | 25 | Outer rim color breaking | 95 |
| 10 | 10 | 50 | Tiger stripe pattern | 97 |
| 11 | 2,500 | 0.4 | Center part color breaking | 98 |
| 12 | 5 | 100 | Transverse color breaking Tiger stripe pattern Dotted pattern | 97 |
| 13 | 1 | 40 | Tiger stripe pattern Dotted pattern | 97 |
| 14 | 100 | 50 | Tiger stripe, Dots | 99 |
| 15 | 1,000 | 10 | Transversal pattern | 97 |
| 16 | 1,000 | 5 | Transversal pattern | 96 |
| 17 | 100 | 5 | Tiger stripe, Dots | 95 |
| 18 | 4,000 | 5 | Tiger stripe, Dots | 96 |
| 19 | 10 | 10 | Transversal pattern Dots | 94 |
| 20 | 1 | 10,000 | Tiger stripe | 95 |
| 21 | 1 | 40,000 | Tiger stripe | 96 |
| 22 | 1 | 40,000 | Tiger stripe | 98 |

| Embodi-ment | Dilution Ratio | Dosage/kg of Body Weight of Plant (mg) | Mode of Variegation | Growth Rate |
|---|---|---|---|---|
| 23 | 1,000 | 5 | Vertical pattern | 97 |
| 24 | 1,000 | 10 | Center part color breaking | 101 |
| 25 | 1,000 | 40 | Vertical patterns | 98 |
| 26 | 100,000 | 2 | Tiger stripe Transversal pattern | 96 |
| 27 | 3,000 | 0.5 | Transveral pattern | 99 |
| 28 | 3,000 | 10 | Dots | 95 |
| 29 | 5,000 | 5 | Vertical pattern | 96 |
| 30 | 200 | 200 | Center part color breaking | 88 |
| 31 | 5 | 100 | Tiger stripe | 98 |
| 32 | 10 | 50 | Dots | 98 |
| 33 | 500 | 100 | Center part color breaking | 99 |
| 34 | 30 | 50 | Center part color breaking | 101 |
| 35 | 20 | 200 | Center part color breaking | 100 |
| 36 | 500 | 200 | Veining pattern | 95 |
| 37 | 10 | 5,000 | Dots | 96 |
| 38 | 20 | 10 | Dots | 95 |
| 39 | 200 | 25 | Outer rim pattern | 98 |
| 40 | 40 | 100 | Center part color breaking, Dots | 95 |
| 41 | 80 | 50 | Center part color breaking | 90 |
| 42 | 20 | 20 | Center part color breaking | 95 |

| Embodiment | Dilution Ratio | Dosage/Kg of Body Weight of Plant (mg) | Mode of Variegation | Growth Rate |
|---|---|---|---|---|
| 43 | 50 | 100 | Center part color breaking | 95 |
| 44 | 200 | 100 | Tiger stripe | 98 |
| 45 | 20,000 | 0.2 | Vertical pattern Center part color breaking | 98 |
| 46 | 200 | 300 | Tiger stripe, Dots | 100 |
| 47 | 20 | 30 | Center part color breaking Veining pattern | 96 |
| 48 | 200 | 50 | Tiger stripe Transversal pattern | 98 |
| 49 | 30 | 30 | Center part color breaking | 96 |
| 50 | 10 | 500 | Tiger stripe | 98 |
| 51 | 10 | 500 | Center part color breaking | 95 |
| 52 | 300 | 15 | Entire leaf whitening | 98 |
| 53 | 50 | 100 | Center part color breaking, Dots | 98 |
| 54 | 10 | 50 | Center part color breaking | 98 |
| 55 | 40 | 250 | Tiger stripe | 95 |
| 56 | 10 | 50 | Center part color breaking | 91 |
| 57 | 1,000 | 10 | Dots | 101 |
| 58 | 30 | 100 | Center part color breaking | 98 |
| 59 | 100 | 150 | Center part color breaking | 98 |
| 60 | 10 | 50 | Veining pattern, Dots | 98 |
| 61 | 200 | 50 | Vertical pattern Center part color breaking | 98 |

| Embodiment | Dilution Ratio | Dosage/kg of Body Weight of Plant (mg) | Mode of Variegation | Growth Rate |
|---|---|---|---|---|
| 62 | 50 | 200 | Tiger stripe | 95 |
| 63 | 100 | 100 | Veining pattern | 98 |
| 64 | 50 | 100 | Veining pattern | 95 |
| 65 | 100 | 50 | Vertical pattern Center part color breaking | 95 |
| 66 | 200 | 40 | Tiger stripe | 95 |
| 67 | 50 | 150 | Center part color breaking | 95 |
| 68 | 3,000 | 50 | Entire leaf whitening Center part color breaking | 98 |
| 69 | 50 | 150 | Veining pattern | 98 |
| 70 | 200 | 100 | Veining pattern | 99 |
| 71 | 10 | 50 | Dots | 96 |
| 72 | 1,000 | 50 | Tiger stripe | 101 |
| 73 | 40 | 40 | Veining Pattern | 96 |
| 74 | 30 | 150 | Center part color breaking | 88 |
| 75 | 50 | 100 | Center part color breaking | 90 |
| 76 | 10,000 | 1 | Center part color breaking | 96 |
| 77 | 200 | 50 | Tiger stripe | 96 |
| 78 | 100 | 80 | Tiger stripe | 98 |
| 79 | 100 | 200 | Center part color breaking | 95 |
| 80 | 500 | 30 | Tiger stripe | 95 |
| 81 | 10 | 50 | Tiger stripe Transversal pattern | 95 |

| Embodiment | Dilution Ratio | Dosage/Kg of Body Weight of Plant (mg) | Mode of Variegation | Growth Rate |
|---|---|---|---|---|
| 82 | 200 | 50 | Center part color breaking | 96 |
| 83 | 1,000 | 10 | Dots | 96 |
| 84 | 30 | 100 | Vertical pattern | 98 |
| 85 | 200 | 100 | Tiger stripe | 95 |
| 86 | 50 | 200 | Vertical pattern Tiger stripe | 96 |
| 87 | 30 | 100 | Dots | 95 |
| 88 | 200 | 200 | Center part color breaking | 88 |
| 89 | 200 | 50 | Veining pattern | 90 |
| 90 | 100 | 300 | Center part color breaking | 95 |
| 91 | 1,000 | 100 | Veining pattern | 95 |
| 92 | 100 | 80 | Center part color breaking | 98 |
| 93 | 200 | 50 | Tiger stripe | 97 |
| 94 | 50 | 100 | Center part color breaking | 97 |
| 95 | 30 | 150 | Veining pattern | 98 |

## Claims

1. A method for producing variegated plants, characterized in that a plant is treated with chlorophyll inhibitors during the growing process of said plant, whereby destroying the chlorophyll in the body of said plant directly or indirectly or inhibiting biosynthesis of said chlorophyll.

2. A method for producing variegated plants as set forth in claim 1, wherein said chlorophyll inhibitors are compounds which inhibit the biosynthesis of chlorophyll itself, causing said plant to be variegated.

3. A method for producing variegated plants as set forth in claim 2, wherein at least one type of compound having an effect to inhibit the biosynthesis of the chlorophyll itself is used, said compound being selected from the group consisting of 4-(2.4-dichlorobenzoyl)-1,3-dimethyl-1H-pyrazol-5-yl p-toluene sulfonate, 3.4-dichlorobenzyl-N-methyl carbamate, 1,1-bis(chlorophenyl)-2.2.2-trichloro-ethanol, a mixture of 0.0-diisopropyl S-(2-phynylsulfonyl-aminoethyl)phosphorodithiate and 4-methoxy-3.3' dimethylbenzophenone, calcium trichloroacetate, ammonium methanearsonate and sodium chlorate.

4. A method for producing variegated plants as set forth in claim 1, wherein said chlorophyll inhibitor is a compound which inhibits the biosynthesis of chlorophyll through the excessive incorporation of boron into plant bodies, causing said plant to be variegated.

5. A method for producing variegated plants as set forth in claim 4, wherein at least one type of compound which inhibits the biosyntheis of chlorophyll through the excessive incorporation of boron into a plant body is used, said compound being selected from the group consisting of sodium pyroborate, sodium octoborate, and sodium metahorate.

6. A method for producing variegated plants as set forth in claim 1, wherein said chlorophyll inhibitor is a compound which inhibits the biosynthesis of chlorophyll through blocking the intake of magnesium, iron, and manganese, causing said plant to be variegated.

7. A method for producing variegated plants as set forth in claim 6, wherein 3-amino-1H-1.2.4-triazole is used as the compound having an effect of inhibiting the incorporation of magnesium, iron, and manganese.

8. A method for producing variegated plants as set forth in claim 1, wherein said chlorophyll inhibitor is a compound which inhibits the biosynthesis of chlorophyll by destroying proteins and amino acids, causing said plant to be variegated.

9. A method for producing variegated plants as set forth in claim 8, wherein at least one type of compound selected from the group consisting of methyl N-(4-nitrophenylsulfonyl) carbamate, methyl-N-(4-amino-phenylsulfonyl) carbamate, isopropyl-N-(3 chlorophenyl) carbamate, 2.4-dinitro-6-secondary butylphenyldimethyl acrylate, dinitromethylheptylphenyl crotonate, a,a,a-trifluoro-2.6-dinitro-N,N-dipropyl-p-tluidine, 4-(methyl-sulfonyl)-2.6-dinitro-N,N-dipropylaniline, 2.4-dinitro-6-secondary butylphenol acetate, 2.2-sodium dichloropropionate, a-(2-naphthoxy) propionanilide, and basic copper chlorideis is used as a compound which inhibits the biosynthesis of chlorophyll by destroying proteins and amino acids.

10. A method for producing variegated plants as set forth in claim 1, wherein said chlorophyll inhibitor is a compound which inhibits the biosynthesis of chlorophyll through abnormal growth of cells by means of hormonal action, causing said plant to be variegated.

11. A method for producing variegated plants as set forth in claim 10, wherein at least one type of compound selected from the group consisting of 2.3.5-trichloro-4-hydroxypyridine, 3.5-dichloro-2.6-difluoro-4-hydroxypyridine, 0-methyl-0-(nitro-4-methylphenyl)-N-isopropylphosphoroamidethiate, 2-methyl-4-chloro-phenoxyacetic acid, dichlorophenoxyacetic acid, a-(2-naphtoxy) propionanilide, 2.2-sodium dichloropropionate, isopropyl-N-(3-chlorophenyl) carbamate, and gibberellin is used as a compound which inhibits biosynthesis of chlorophyll through abnormal growth of cells by means of hormonal action.

12. A method for producing variegated plants as set forth in claim 1, wherein said chlorophyll inhibitor is a compound which inhibits formation of carotinoid which inhibits photolysis of chlorophyll, causing variegation is said plant.

13. A method for producing variegated plants as set forth in claim 12, wherein at least one type of compound selected from the group consisting of 4-methoxy-3.3'-dimethylbenzophenone, 2-[4-(2.4-dichlorobenzoyl)-1.3-dimethylpyrazol-5-yloxy] acetophenone, 4-chloro-5-methylamino-2-(3-trifluoromethylphenyl) pyridazin-3(2H)-one, 4-chloro-5-dimethylamino-2-(3-trifluoromethylphenyl) pyridazin-3(2H)-one, 2.3.5-trichloro-4-hydoxypyridine, and 1-methyl-3-phenyl-5-(3-trifluoromethylphenyl) pyridin 1-4(1H)-one is used as a compound which inhibits the formation of carotinoid which prevents photolysis of chlorophyll.

14. A method for producing variegated plants as set forth in claim 1, wherein said chlorophyll inhibitor is a compound which inhibits the biosynthesis of chlorophyll by blocking the electron transer system, causing said plant to be variegated.

15. A method for producing variegated plants as set forth in claim 14, wherein 1.2-dimethyl-3.5-diphenyl-1H-pyrazolium methyl sulfate is used as a compound that inhibits the biosynthesis of chlorophyll by blocking the electron transfer system.

16. A method for producing variegated plants as set forth in claim 1, wherein said chlorophyll inhibitor is used in the range of 0.0001 to 100,000 mg per kg of the body weight of said plant.

17. A method for producing variegated plants as set forth in claim 1, wherein said chlorophyll inhibitor is used in a form selected from the group consisting of a liquid, particle, and powder, containing said inhibiting agent.

18. A method for producing variegated plants as set forth in claim 1, wherein said chlorophyll inhibitor is sprayed on parts of said plant, which are located on or above the ground, such as a leaf blade, or poured onto the root and soil, or buried in the soil, or injected into the soil and the body of said plant.

19. A method for producing variegated plants as set forth in claim 1, wherein said growing process is a germination stage or a leaf growing stage of said plant.

20. A method for producing variegated plants as set forth in claim 1, wherein not less than one region selected from a germ, leaf, stem and root of said plant are treated with a prohibitor.

21. A method for producing variegated plants as set forth in claim 1, wherein said is a foliage plant which belongs to one selected from the group consisting of Liliaceae, Agavaceae, Araceae, Myrsinaceae, Araliaceae, Musaceae, Moraceae, Bombacaceae, Bromeliaceae, Marantaceae, Enphorbiaceae, Zingiberaceae, Orchidaceae, Amaryllidaceae, Pandanaceae, Cyperaceae, Palmae (Coryphaceae), Polypodiaceae, Aspleniaceae, Marattiaceae, Pteridiaceae, Davalliaceae, Cyatheaceae, Dryopteraceae, Araucariaceae, Cycadaceae, Cupressaceae, Podocarpaceae, Urticaceae, Nyctaginaceae, Sarcandraceae, Hypericaceae, Sarranceniaceae, Begoniaceae, Asteraceae, Cactaceae, Schisandraceae, Lauraceae, Piperaceae, Crassulaceae, Saxifragaceae, Rutaceae, Aquifoliaceae, Vitaceae, Sterculiaceae, Passifloraceae, Caricaceae, Myrtaceae, Melastomaceae, Rhizophoraceae, Apocynaceae, Asclepiadaceae Rubiaceae, Verbenaceae, Lamiaceae, Bignoniaceae, Acanthaceae, Gesneriaceae, Caprifoliaceae, Campanulaceae, Goodeniaceae, Iridaceae, Commelinaceae, Gramineae (Poaceae), Bambusa in Gramineae Bamnusaceae, Equisetaceae, Primulaceae, Pittosporaceae, Oak in Fagaceae, Gentianaceae, Pinaceae, Buxaceae, and Myricaceae.

22. A method for producing variegated plants as set forth in claim 1, wherein said variegation is effected to a plant, such as Yucca and Chamaedorea planted in a pot, by pouring a solution of said chlorophyll inhibitor onto the roots of said plant or into the soil, by burying a powder of said chlorophyll inhibitor around the roots of said plant or onto the soil, by spraying said powder of said chlorophyll inhibitor onto the surface of the leaves of said plant, or by injecting said chrolophyll inhibitor into the body of said plant.

23. A method for producing variegated plants as set forth in claim 1, wherein said variegation is effected to an outdoor plant, such as Rhodea japonica (Rohdea) and Adiantum, by irrigating the soil with said chrolophyll inhibitor, by burying said chlorophyll inhibitor into the soil, by injecting said chlorophyll inhibitor into the body of said plant or in the soil, or by spraying said chlorophyll inhibitor onto the surface of the leaves of said plant.

24. A method for producing variegated plants as set forth in claim 1, wherein, by utilizing the respective effects of various types of chlorophyll inhibitors, said variegation a whitening color breaking, yellowing color breaking, color breaking with a clear boundary line dividing the chlorophyll portion and the variegated portion, color breaking with a sprinkly boundary between the chlorophyll portion and the variegated portion, light white and light yellow color breakings, color breaking with wide variegated portions, or color breaking with narrow variegated portion.

25. A method for producing variegated plants as set forth in claim 1, wherein said variegation is effected by utilizing respective characteristics of various types of plants such as veining and capability to absorb chemicals from leaf surface so that variegation modes of tiger stripe color breaking, vertical color breaking, transverse color breaking, center part color breaking, dotted color breaking, veining color breaking, entire leaf color breaking, whitening color breaking, or yellowing color breaking is produced.

26. A method for producing variegated plants as set forth in claim 1, wherein said variegation is effected by using various types of treating methods including spraying, irrigation, injection, and burial, with the variegation producing compounds to produce a zigzagged boundary line between the chlorophyll portion and the variegated portion, a straight boundary line between the chlorophyll portion and the variegated portion, a variegated area with absolutely no chlorophyll portion remaining in the variegated area, or a chlorophyll portion remaining in the form of a cloud-shape, dots, or vertical stripes.

## Patentansprüche

1. Ein Verfahren zur Herstellung scheckiger Pflanzen, dadurch **gekennzeichnet** , daß eine Pflanze mit Chlorophyllinhibitoren während des Wachstumsprozesses der Pflanze behandelt wird, wodurch das Chlorophyll in dem Körper der Pflanze direkt oder indirekt zerstört oder die Biosynthese des Chlorophylls behindert wird.

2. Ein Verfahren zur Herstellung scheckiger Pflanzen nach Anspruch 1, bei dem die Chlorophyllinhibitoren Verbindungen sind, die die Biosynthese des Chlorophylls selbst behindern, wodurch die Pflanze scheckig wird.

3. Ein Verfahren zur Herstellung scheckiger Pflanzen nach Anspruch 2, bei dem mindestens eine Verbindungstype, die selbst eine Veränderungswirkung der Biosynthese des Chlorophylls hat, benutzt wird, wobei die Verbindung aus der Gruppe ausgewählt wird, die aus 4-(2.4-Dichlorbenzoyl) -1,3-Dimethyl-1H-Pyrazol-5-yl p-Toluensulfonat, 3.4-Dichlorbenzyl-N-Methylcarbamat, 1,1-bis (Chlorphenyl)-2.2.2-Dichlor-Ethanol, einer Mischung aus 0.0-Diisopropyl S-(2-Phynylsulonyl-Aminoethyl) Phosphordithioat und 4-Methoxy-3.3′ Dimethylbenzophenon, Calciumtrichloracetat, Ammoniummethanarsonat und Natriumchlorat besteht.

4. Ein Verfahren zur Herstellung scheckiger Pflanzen nach Anspruch 1, bei dem der Chlorophyllinhibitor eine Verbindung ist, die die Biosynthese von Chorophyll durch die heftige Aufnahme von Bor in die Pflanzenkörper behindert, wodurch die Pflanze scheckig wird.

5. Ein Verfahren zur Herstellung scheckiger Pflanzen nach Anspruch 4, bei dem mindestens eine Verbindungstype benutzt wird, die die Biosynthese von Chlorophyll durch die heftige Aufnahme von Bor in einen Pflanzenkörper behindert, wobei die Verbindung aus der Gruppe ausgewählt wird, die aus Natriumpyroborat, Natriumoctaborat und Natriummetaborat besteht.

6. Ein Verfahren zur Herstellung scheckiger Pflanzen nach Anspruch 1, bei dem der Chlorophyllinhibitor eine Verbindung ist, die die Biosynthese von Chlorophyll durch die Blockierung der Aufnahme von Magnesium, Eisen und Mangan unterbindet, wodurch die Pflanze scheckig wird.

7. Ein Verfahren zur Herstellung scheckiger Pflanzen nach Anspruch 6, bei dem 3-Amino-1H-1.2.4-Triazol benutzt wird als die Verbindung, die die Wirkung der Unterbindung der Aufnahme von Magnesium, Eisen und Mangan hat.

8. Ein Verfahren zur Herstellung scheckiger Pflanzen nach Anspruch 1, bei dem der Chlorophyllinhibitor eine Verbindung ist, die die Biosynthese von Chlorophyll durch Zerstörung von Proteinen und Aminosäuren verhindert, wodurch die Pflanze scheckig wird.

9. Ein Verfahren zur Herstellung scheckiger Pflanzen nach Anspruch 8, bei dem mindestens eine Verbindungstype, die aus der Gruppe ausgewählt ist, die aus Methyl N-(4-Nitrophenylsulfonyl)Carbamat, Methyl-N-(4-Amino-Phenylsulfonyl) Carbamat, Isopropyl-N-(3 Chlorphenyl)Carbamat, 2.4-Dinitro-6 Sekundärbutylphenyldimethyl Acrylat, Dinitromethylheptylphenyl Crotonat, a.a.a-Trifluor-2.6-Dinitro-N,N-Dipropyl-p-Toluidin, 4-(Methyl-Sulfonyl)-2.6-Dinitro-N, N-Dipropylanilin, 2.4-Dinitro-6-Sekundärbutylphenol Acetat, 2.2-Natriumdichlorpropionat, a-(2-Naphthoxy) Propionanilid, und basisches Kupferchlorid besteht, als Verbindung benutzt wird, die die Biosynthese von Chorophyll durch Zerstören von Proteinen und Aminosäuren verhindert.

10. Ein Verfahren zur Herstellung scheckiger Pflanzen nach Anspruch 1, bei dem der Chlorophyllinhibitor eine Verbindung ist, die die Biosynthese von Chlorophyll durch abnormales Zellwachstum mit Hilfe von hormonal wirkenden Mitteln verhindert, wodurch die Pflanze scheckig wird.

11. Verfahren zur Herstellung scheckiger Pflanzen nach Anspruch 10, bei dem mindestens eine Verbindungstype, die aus der Gruppe ausgewählt ist, die aus 2.3.5.-Dichlor-4-Hydroxy-Pyridin, 3.5-Dichlor-2.6-Difluor-4-Hydroxypyridin, 0-Methyl-0-(Nitro-4-Methylphenyl)-N-Isopropylphosphoramidthioat, 2-Methyl-4-Chlor-phenoxysaure Säure, dichlorphenoxysaure Säure, a-(2-Naphthoxy) Propionanilid, 2.2-Natriumdichlorpropionat, Isopropyl-N-(3-Chlorphenyl) Carbamat und Gibberellin besteht, als Verbindung benutzt wird, die die Synthese von Chlorophyll durch abnormales Zellenwachstum mit Hilfe hormonal wirkender Mittel verhindert.

12. Ein Verfahren zur Herstellung scheckiger Pflanzen nach Anspruch 1, bei dem der Chlorophyllinhibitor eine Verbindung ist, die die Bildung von Carotinoid verhindert, das die Photolyse of Chlorophyll verhindert, wodurch in der Pflanze eine Scheckigkeit entsteht.

13. Verfahren zur Herstellung scheckiger Pflanzen nach Anspruch 12, bei dem mindestens eine Verbindungstype, die aus der Gruppe ausgewählt ist, die aus 4-Methoxy-3.3'-Dimethyl-Benzophenon, 2-[4-(2.4-Dichlorbenzoyl)-1.3-Dimethylpyrazol-5-Yloxy ] Acetophenon, 4-Chlor-5-Methylamino-2-(3-Trifluormethylphenyl) Pyridazin-3 (2H)-eins, 4-Chlor-5-Dimethylamino-2-(3-Trifluormethylphenyl) Pyridazin-3(2H) -eins, 2. 3.5-Trichlor-4-Hydroxypyridin und 1-Methyl-3-Phenyl-5-(3-Trifluormethylphenyl) Pyridin 1-4 (1H)-eins besteht, benutzt wird als eine Verbindung, die die Bildung von Carotinoid verhindert, was die Photolyse von Chlorophyll unterbindet.

14. Ein Verfahren zur Herstellung scheckiger Pflanzen nach Anspruch 1, bei dem der Chlorophyllinhibitor eine Verbindung ist, die die Biosynthese von Chlorophyll durch Blockierung des Elektronentransfersystems verhindert, wodurch die Pflanze scheckig wird.

15. Verfahren zur Herstellung scheckiger Pflanzen nach Anspruch 14, bei dem 1.2-Dimethyl-3.5-Diphenyl-1H-Pyrazoliummethylsulfat benutzt wird als eine Verbindung, die die Biosynthese von Chlorophyll durch Blockieren des Elektronentransfersystems verhindert.

16. Ein Verfahren zur Herstellung scheckiger Pflanzen nach Anspruch 1, bei dem der Chlorophyllinhibitor im Bereich von 0,0001 bis 100,000 mg pro kg des Körpergewichtes der Pflanze eingesetzt wird.

17. Ein Verfahren zur Herstellung scheckiger Pflanzen nach Anspruch 1, bei dem der Chlorophyllinhibitor in einer Form benutzt wird, die aus der Gruppe ausgewählt wird, die aus einer Flüssigkeit, einem Partikel und einem Pulver besteht, die bzw. das die Inhibitorreagenz enthält.

18. Ein Verfahren zur Herstellung scheckiger Pflanzen nach Anspruch 1, bei dem der Chlorophyllinhibitor auf Teile der Pflanze gesprüht wird, die auf dem oder über dem Boden angeordnet sind, wie z. B. Blätter, auf die Wurzel und den Boden geschüttet wird, in den Boden eingegraben wird oder in den Boden oder den Körper der Pflanze eingespritzt wird.

19. Ein Verfahren zur Herstellung scheckiger Pflanzen nach Anspruch 1, bei dem der Wachstumsprozess eine Keimphase oder eine Blattwachstumsphase der Pflanze ist.

20. Ein Verfahren zur Herstellung scheckiger Pflanzen nach Anspruch 1, bei dem nicht weniger als eine Region mit einem Prohibitor behandelt wird, die aus einem Keim, einem Blatt, einem Stamm und einer Wurzel der Pflanze ausgewählt ist.

21. Ein Verfahren zur Herstellung scheckiger Pflanzen nach Anspruch 1, bei dem die Pflanze eine Blattpflanze ist, die zu einer der Gruppe gehört, die aus der Gruppe ausgewählt ist, die aus Liliaceae, Agavaceae, Araceae, Myrsinaceae, Araliaceae, Musaceae, Moraceae, Bombacaceae, Bromeliaceae, Marantaceae, Enphorbiaceae, Zingiberaceae, Orchidaceae, Amaryllidaceae, Pandanaceae, Cyperaceae, Palmae (Coryphaceae), Polypodiaceae, Aspleniaceae, Marattiaceae, Pteridiaceae, Davalliaceae, Cyatheaceae, Dryopteraceae, Araucariaceae, Cycadaceae, Cupressaceae, Podocarpaceae, Urticaceae, Nyctaginaceae, Sarcandraceae, Hypericaceae, Sarraceniaceae, Begoniaceae, Asteraceae, Cactaceae, Schisandraceae, Lauraceae, Piperaceae, Crassulaceae, Saxifragaceae, Rutaceae, Aquifoliaceae, Vitaceae, Sterculiaceae, Passifloraceae, Caricaceae, Myrtaceae, Melastomaceae, Rhizophoraceae, Apocynaceae, Asclepiadaceae, Rubiaceae, Verbenaceae, Lamiacea, Bignoniaceae, Acanthaceae, Gesneriaceae, Caprifoliaceae, Campanulaceae, Goodeniaceae, Iridaceae, Commelinaceae, Gramineae (Poaceae), Bambusa in Gramineae Bamnusaceae, Equisetaceae, Primulaceae, Pittosporaceae, Oak in Fagaceae, Gentianaceae, Pinaceae, Buxaceae und Myricaceae besteht.

22. Ein Verfahren zur Herstellung scheckiger Pflanzen nach Anspruch 1, bei dem die Scheckenbildung an einer Pflanze wie z. B. in einen Topf eingepflanzte Yucca und Chamaedorea durch Gießen einer Lösung des Chlorophyllinhibitors auf die Wurzeln der Pflanze oder in den Boden, durch Eingraben von Pulver des Chlorophyllinhibitor um die Wurzeln der Pflanze oder Auflegen auf den Boden, durch Aufsprühen des Puders des Chlorophyllinhibitors auf die Oberfläche der Blätter der Pflanze oder durch Einspritzen des Chlorophyllinhibitors in den Körper der Pflanze bewirkt wird.

23. Ein Verfahren zur Herstellung scheckiger Pflanzen nach Anspruch 1, bei dem die Scheckenbildung an einer Freiluftpflanze wie z. B. der Rhodea japonica und Adiantum durch Benetzen des Bodens mit dem Chlorophyllinhibitor, durch Eingraben des Chlorophyllinhibitors in den Boden, durch Einspritzen des Chlorophyllinhibitors in den Körper der Pflanze oder den Boden oder durch Aufsprühen des Chlorophyllinhibitors auf die Oberfläche der Blätter bewirkt wird.

24. Ein Verfahren zur Herstellung scheckiger Pflanzen nach Anspruch 1, bei dem durch die Benutzung der jeweiligen Effekte von unterschiedlichen Typen von Chlorophyllinhibitoren die Scheckenbildung eine Weißfärbung, eine Gelbfärbung, einen Farbübergang mit klaren Grenzlinien, die die Chlorophyllabschnitte und die scheckigen Abschnitte voneinander trennen, eine Farbunterscheidung mit einer gesprenkelten Grenze zwischen den Chlorophyllabschnitten und den gescheckten Abschnitten, eine leichte Weiß- und leichte Gelbfärbung, eine Farbunterscheidung mit weiten scheckigen Abschnitten oder eine Farbabsetzung mit schmalen scheckigen Abschnitten ist.

25. Ein Verfahren zur Herstellung scheckiger Pflanzen nach Anspruch 1, bei dem die Scheckenbildung durch den Einsatz der entsprechenden Charakteristiken verschiedener Typen von Pflanzen wie z. B. Äderung und die Fähigkeit, Chemikalien von Blattoberflächen zu absorbieren, bewirkt wird, so daß Scheckenbildungen in der Art von Tigerstreifen, vertikalen Farbabsetzungen, querverlaufenden Farbabsetzungen, zentrierten Farbabsetzungen, gepunkteten Farbabsetzungen, adrigen Farbabsetzungen, Farbbildungen ganzer Blätter, Weißung oder Gelbbildung hervorgebracht wird.

26. Ein Verfahren zur Herstellung scheckiger Pflanzen nach Anspruch 1, bei dem die Scheckenbildung durch den Einsatz unterschiedlicher Typen von Behandlungsmethoden einschließlich Sprühen, Befeuchten, Einspritzen und Eingraben bewirkt wird, wobei die die Scheckenbildung hervorbringenden Verbindungen eine gezackte Grenzlinie zwischen dem Chlorophyllabschnitt und dem gescheckten Abschnitt, eine gerade Grenzlinie zwischen dem Chlorophyllabschnitt und dem gescheckten Abschnitt, eine gescheckte Zone, bei der absolut keine Chlorophyllzone in der farbveränderten Zone verbleibt, oder einen Chlorophyllabschnitt produziert, der in Form einer Wolke, von Punkten oder vertikalen Streifen verbleibt.

## Revendications

1. Procédé pour la production de plantes panachées, caractérisé en ce qu'une plante est traitée à l'aide d'inhibiteurs de la chlorophylle pendant le processus de croissance de cette plante, ce qui détruit directement ou indirectement la chlorophylle dans le corps de la plante ou inhibe la biosynthèse de la chlorophyle.

2. Procédé pour produire des plantes panachées conforme à la revendication 1 selon lequel les inhibiteurs de la chlorophylle sont des composés qui inhibent la biosynthèse de la chlorophylle elle-même ce qui fait que la plante est panachée.

3. Procédé pour la production de plantes panachées conforme à la revendication 2 selon lequel au moins un type de composé ayant un effet d'inhibition de la biosynthèse de la chlorophylle elle-même est utilisé, ce composé étant choisi dans le groupe composé de : sulfonate de 4-(2,4-dichlorenzoyl)-1,3-diméthyl -1H-pyrazol-5-yl-p-toluène, 3,4-dichlorenzyl-N-méthyl-carbamate, 1,1-bis(chlorophényl)-2,2,2-trichloroéthanol, un mélange de 0,0-diisopropyl-S-(2-phynylsulfonyl-aminoéthyl)phosphorodithioate et de 4-méthoxy-3,3′-diméthylbenzophénone, trichloroacétate de calcium , méthane-arsonate d'ammonium et chlorate de sodium.

EP 0 257 845 B1

4. Procédé pour la production de plantes panachées conforme à la revendication 1 selon lequel l'inhibiteur de la chlorophylle est un composé qui inhibe la biosynthèse de la chlorophylle au moyen de l'incorporation excessive de bore dans le corps de la plante, faisant que cette plante est panachée.

5. Procédé pour la production de plantes panachées conforme à la revendication 4 selon lequel au moins un type de composés qui inhibe la biosynthèse de la chlorophylle au moyen de l'incorporation excessive de bore au corps de la plante est utilisé, ce composé étant choisi dans le groupe composé de : pyroborate de sodium, octaborate de sodium et métaborate de sodium.

6. Procédé pour la production de plantes panachées conforme à la revendication 1 selon lequel l'inhibiteur de la chlorophylle est un composé qui inhibe la biosynthèse de la chlorophylle au moyen du blocage de l'incorporation du magnésium, du fer et du manganèse, faisant que la plante est panachée.

7. Procédé pour la production de plantes panachées conforme à la revendication 6 selon lequel le 3-amino-1H- 1,2, 4 -triazole est utilisé comme composé ayant un effet d'inhibition sur l'incorporation du magnésium, du fer et du manganèse.

8. Procédé pour la production de plantes panachées conforme à la revendication 1 selon lequel l'inhibiteur de la chlorophylle est un composé qui inhibe la biosynthèse de la chlorophylle par la destruction de protéines et d'amino-acides, faisant que la plante est panachée.

9. Procédé pour la production de plantes panachées conforme à la revendication 8 , selon lequel au moins un type de composé choisi dans le groupe composé de : méthyl-N-(4-nitrophénylsulfonyl)-carbamate, méthyl-N-(4-amino-phynylsulfonyl)-carbamate, isopropyl-N-(3-chlorphényl)-carbamate, 2,4-dinitro-6-sec. butyl-phényl-diméthylacrylate, dinitrométhylheptylphényl-crotonate, a,a,a-trifluoro-2,6-dinitro-N,N-dipropyl-p-toluidine, 4-(méthyl-sulfonyl)-2,6-dinitro-N,N-dipropylaniline, 2,4-dinitro-6-sec.-butyl-phénolacétate, 2,2-dichloropropionate de sodium, a-(2-naphthoxy)-propionanilide et le chlorure de cuivre basique,est utilisé comme composé qui inhibe la biosynthèse de la chlorophylle par la destruction de protéines et d'amino-acides.

10. Procédé pour la production de plantes panachées conforme à la revendication 1 selon lequel l'inhibiteur de la chlorophylle est un composé qui inhibe la biosynthèse de la chlorophylle au moyen d'une croissance anormale des cellules par l'intermédiaire d'une action hormonale faisant que la plante est panachée.

11. Procédé pour la production de plantes panachées conforme à la revendication 10 selon lequel au moins un type de composé choisi dans le groupe composé de : 2,3,5-trichloro-4-hydroxy-pyridine, 3,5-dichloro-2,6-difluoro-4,hydroxy-pyridine, 0-méthyl-0-(nitro-4-méthylphényl)-N-isopropylphosphoroamidethioate, acide 2-méthyl-4-chloro-phénoxyacétique, acide dichlorophénoxyacétique, a-(2-naphtoxy)-propionanilide, 2,2-dichloropropionate de sodium, isopropyl-N-(3-chlorophényl)-carbamate et la gibberelline est utilisée comme composé qui inhibe la biosynthèse de la chlorophylle au moyen d'une croissance anormale des cellules par une action hormonale.

12. Procédé pour la production de plantes panachées conforme à la revendication 1, selon lequel l'inhibiteur de la chlorophylle est un composé qui inhibe la formation du carotinoïde qui inhibe lui-même la photolyse de la chlorophylle, ce qui provoque le panachage de la plante.

13. Procédé pour la production de plantes panachées conforme à la revendication 12, selon lequel au moins un type de composé choisi dans le groupe composé de : 4-méthoxy-3,3'-diméthyl-benzophénone, 2-{4-(2,4-dichlorobenzoyl)-1,3-diméthylpyrazol-5-yloxy}-acétophénone, 4-chloro-5-méthylamino-2-(3-trifluorométhylphényl)-pyridazin-3(2H)-one , 4-chloro-5-diméthylamino-2-(3-trifluorométhylphényl)-pyridazin-3(2H)-one, 2,3,5-trichloro-4-hydroxypyridine et 1-méthyl-3-phényl-5--(3-trifluorométhylphényl)-pyridin-1-4(1H)-one est utilisé comme composé qui inhibe la formation du carotinoïde ce qui empêche la photolyse de la chlorophylle.

14. Procédé pour la production de plantes panachées conforme à la revendication 1 selon lequel l'inhibiteur de la chlorophylle est un composé qui inhibe la biosynthèse de la chlorophylle par le blocage du système de transfert d'électrons, faisant que la plante est panachée.

15. Procédé pour la production de plantes panachées conforme à la revendication 14 selon lequel du méthylsulfate de 1,2-diméthyl-3,5-diphényl-1H-pyrazolium est utilisé comme composé qui inhibe la biosynthèse de la chlorophylle par le blocage du système de transfert d'électrons.

16. Procédé pour la production de plantes panachées conforme à la revendication 1 selon lequel l'inhibiteur de la chlorophylle est utilisé dans la gamme de 0,0001 à 100.000 mg par kilo du poids du corps de la plante.

17. Procédé pour la production de plantes panachées conforme à la revendication 1 selon lequel l'inhibiteur de la chlorophylle est utilisé sous une forme choisie dans le groupe composé d'un liquide, de particules et de poudre contenant l'agent inhibiteur.

18. Procédé pour la production de plantes panachées conforme à la revendication 1 selon lequel l'inhibiteur de la chlorophylle est pulvérisé sur des parties de la plante qui sont situées sur le sol ou au-dessus du sol comme une feuille ou versé sur les racines et le sol ou enterré dans le sol ou injecté dans le sol et dans le corps de la plante.

19. Procédé pour la production de plantes panachées conforme à la revendication 1 selon lequel le processus de croissance est un stade de germination ou un stade de développement des feuilles de la plante.

20. Procédé pour la production de plantes panachées conforme à la revendication 1 selon lequel pas moins d'une région choisie entre un germe, une feuille, une tige et une racine de la plante est traitée à l'aide d'un inhibiteur.

21. Procédé pour la production de plantes panachées conforme à la revendication 1 selon lequel cette plante est une plante à feuillage qui appartient à l'une choisie dans le groupe composé de : Liliaceae, Agavaceae, Araceae, Myrsinaceae, Araliaceae, Musaceae, Moraceae, Bombacaceae, Bromeliaceae, Marantaceae, Enphorbiaceae,Zingiberaceae, Orchidaceae, Amaryllidaceae, Pandanaceae, Cyperaceae, Palmae (Coryphaceae), Polypodiaceae, Aspleniaceae, Marattiaceae, Pteridiaceae, Davalliaceae, Cyatheaceae, Dryopteraceae, Araucariaceae, Cycadaceae, Cupressaceae, Podocarpaceae, Urticaceae, Nyctaginaceae, Sarcandraceae, Hypericaceae, Sarraceniaceae, Begoniaceae, Asteraceae, Cactaceae, Schisandraceae, Lauraceae, Piperaceae, Crassulaceae, Saxifragaceae, Rutaceae, Aquifoliaceae, Vitaceae, Sterculiaceae, Passifloraceae, Caricaceae, Myrtaceae, Melastomaceae, Rhizophoraceae, Apocynaceae, Asclepiadaceae, Rubiaceae, Verbenaceae, Lamiaceae, Bignoniaceae, Acanthaceae, Gesneriaceae, Caprifoliaceae, Campanulaceae, Goodeniaceae, Iridaceae, Commelinaceae, Gramineae (Poaceae), Bambusa en Gramineae Bamnusaceae, Equisetaceae, Primulaceae, Pittosporaceae, Oak en Fagaceae, Gentianaceae, Pinaceae, Buxaceaen et Myricaceae.

22. Procédé pour la production de plantes panachées conforme à la revendication 1 selon lequel le panachage est effectué sur une plante comme un Yucca et un Chamaecorea plantée dans un pot, par versement d'une solution d'un inhibiteur de chlorophylle sur les racines de cette plante ou sur le sol, d'un enfouissement d'une poudre d'inhibiteur de chlorophylle autour des racines de la plante ou dans le sol, par pulvérisation de la poudre de l'inhibiteur de chlorophylle à la surface des feuilles de la plante ou par injection d'inhibiteur de chlorophylle dans le corps de la plante.

23.Procédé pour la production de plantes panachées conforme à la revendication 1 selon lequel le panachage est effectué sur une plante extérieure comme Rhodea japonica (Rohdea) et Adiantum, par irrigation du sol à l'aide de l'inhibiteur de chlorophylle, enfouissement dans le sol de l'inhibiteur de chlorophylle, par injection dans le corps de la plante ou dans le sol de l'inhibiteur de chlorophylle et par pulvérisation de l'inhibiteur de chlorophylle à la surface des feuilles de la plante.

24. Procédé pour la production de plantes panachées conforme à la revendication 1 selon lequel par l'emploi des effets respectifs des divers types d'inhibiteurs de chlorophylle, le panachage est un changement de la couleur vers le blanc un changement de la couleur vers le jaune, un changement de la couleur avec des limites claires divisant la partie à chlorophylle et la partie panachée, un changement de la couleur avec des zones parsemées de la partie avec chlorophylle et de la partie panachée, des changements de la couleur du type blanc pâle et jaune pâle, un changement de la couleur avec de grandes parties panachées, ou un changement de la couleur avec des parties panachées étroites.

25. Procédé pour la production de plantes panachées conforme à la revendication 1 selon lequel le panachage est effectué par l'utilisation des caractéristiques respectives de divers types de plantes comme le nervurage et la capacité d'absorber des produits chimiques par la surface des feuilles, de sorte que des modes de panachage du type changement de couleurs par bandes comme celle d'un tigre, changement de couleur en sens vertical, changement de couleur en sens transversal, changement de couleur dans des zones centrales, changement de couleur par points, changement de couleur par nervurage, changement de couleur de la feuille entière, changement de couleur par blanchiment, ou changement de couleur par jaunissement, sont produits.

26. Procédé pour la production de plantes panachées conforme à la revendication 1 selon lequel le panachage est effectué par l'emploi de divers types de méthodes de traitement incluant la pulvérisation, l'irrigation, l'injection, l'enfouissement, à l'aide de composés produisant le panachage pour l'obtention d'une limite en zigzag entre la partie avec chlorophylle et la partie panachée, avec une ligne droite entre la partie avec chlorophylle et la partie panachée, avec une surface panachée ayant une partie absolument sans chlorophylle restant dans la zone panachée ou une partie avec chlorophylle restant sous une forme ayant la configuration de nuage, de points, ou de bandes verticales.

Fig 1 (a)

Fig 1 (b)

Fig. 2 (a)

Fig. 2 (b)

Fig 3 (a)

Fig 3 (b)

Fig 4 (a)

Fig 4 (b)

Fig 5 (a)

Fig 5 (b)

Fig 6 (a)

Fig 6 (b)

Fig. 1

Fig. 5

Fig 9

Fig 10

Fig 11

Fig 12

Fig 13 (a)

Fig 13 (b)

Fig 13 (c)